# EUROPEAN PATENT SPECIFICATION

(11) **EP 0 885 186 B1**
(45) Date of publication and mention of the grant of the patent: **26.03.2003**
(21) Application number: 96941032.3
(22) Date of filing: 26.11.1996
(51) Int. Cl.: C07C 237/22, A61K 31/195

(54) **AMINO ACID DERIVATIVES, PHARMACEUTICAL COMPOSITIONS CONTAINING THESE COMPOUNDS AND PROCESSES FOR PREPARING THEM**
DERIVATE VON AMINOSÄUREN, SIE ENTHALTENDE PHARMAZEUTISCHE ZUSAMMENSETZUNGEN SOWIE DEREN HERSTELLUNSGVERFAHREN
DERIVES D'ACIDES AMINES, COMPOSITIONS PHARMACEUTIQUES CONTENANT CES COMPOSES ET PROCEDES DE PREPARATION DE CEUX-CI

(30) Priority: 30.11.1995 DE 19544687
(43) Date of publication of application: 23.12.1998
(73) Proprietor: Dr. Karl Thomae GmbH, D-88397 Biberach (DE)
(72) Inventor: ENGEL, Wolfhard, D-88400 Biberach (DE); EBERLEIN, Wolfgang, D-88400 Biberach (DE); RUDOLF, Klaus, D-88400 Biberach (DE); DOODS, Henri, D-88447 Warthausen (DE); WIELAND, Heike-Andrea, D-88400 Biberach (DE); WILLIM, Klaus-Dieter, D-88454 Hochdorf (DE); ENTZEROTH, Michael, D-88447 Warthausen (DE); WIENEN, Wolfgang, D-88400 Biberach (DE)
(74) Representative: Laudien, Dieter, Dr.
(86) International application number: EP9605222
(87) International publication number: WO97019911

(56) References cited:
- WO-A-94/17035
- DE-A- 4 301 452

## Description

The present invention relates to new amino acid derivatives selected from the group consisting of
(1) (R)-N-[[4-(Aminocarbonylaminomethyl)phenyl]methyl]-N²-(diphenylacetyl)-argininamide,
(2) (R,S)-N⁵-(Aminoiminomethyl)-N²-(diphenylacetyl)-N-[(4-hydroxyphenyl)methyl]-N⁵-methyl-ornithinamide,
(3) (R)-N-[[4-(Aminocarbonylmethyl)phenyl]methyl]-N²-(diphenylacetyl)-argininamide,
(4) (R)-N²-(Diphenylacetyl)-N-[[4-(methylaminocarbonyl-aminomethyl)phenyl]methyl]-argininamide,
(5) (R)-N²-(Diphenylacetyl)-N-[[4-(ethylaminocarbonylamino-methyl)phenyl]methyl]-argininamide,
(6) (R)-N-[[4-(Aminocarbonylaminomethyl)phenyl]methyl]-N²-[bis-(4-methoxyphenyl)-acetyl]-argininamide,
(7) (R)-N²-(Diphenylacetyl)-N-[[4-(ethoxycarbonylmethylamino-carbonylaminomethyl)-phenyl]methyl]-argininamide,
(8) (R)-N-[[4-(Aminocarbonylaminomethyl)phenyl]methyl]-N²-[bis-(4-fluorophenyl)-acetyl]-argininamide,
(9) (R)-N-[[4-(Aminocarbonylaminomethyl)phenyl]methyl]-N²-[bis-(4-chlorophenyl)-acetyl]-argininamide,
(9) (R)-N-[[4-(Aminocarbonylaminomethyl)phenyl]methyl]-N²-[bis-(4-chlorophenyl)-acetyl]-argininamide,
(10) (R)-N-[[4-(Aminocarbonylaminomethyl)phenyl]methyl]-N²-[bis-(4-hydroxyphenyl)-acetyl]-argininamide,
(11) (R)-N-[[4-(Aminocarbonylaminomethyl)phenyl]methyl]-N²-[bis-[4-(methoxycarbonylmethoxy)phenyl]acetyl]-argininamide and
(12) (R)-N-[[4-(Aminocarbonylaminomethyl)phenyl]methyl]-N²-[bis-[4-(hydroxycarbonylmethoxy)phenyl]acetyl]-argininamide
and the salts thereof, particularly the physiologically acceptable salts thereof with inorganic or organic acids or bases, pharmaceutical compositions containing these compounds, the use thereof and processes for preparing the pharmaceutical compositions.

Amino acid derivatives of related structure possessing NPY-antagonist properties suitable for treatment of inter alia cardiovascular diseases, obesity and diabetes are already disclosed in WO 94/17035.

The compounds according to the invention have valuable pharmacological properties based on their selective NPY-antagonistic properties.

The compounds according to the invention may be converted into the physiologically acceptable salts thereof with inorganic or organic acids, particularly for pharmaceutical use. Suitable acids for this purpose include, for example, hydrochloric acid, hydrobromic acid, phosphoric acid, nitric acid, sulphuric acid, methanesulphonic acid, p-toluenesulphonic acid, acetic acid, fumaric acid, succinic acid, lactic acid, mandelic acid, malic acid, citric acid, tartaric acid or maleic acid.

Moreover, the new compounds according to the invention, if they contain a carboxy group, may if desired subsequently be converted into the addition salts thereof with inorganic or organic bases, particularly for pharmaceutical use into the physiologically acceptable addition salts thereof. Suitable bases include, for example, sodium hydroxide, potassium hydroxide, ammonia, cyclohexylamine, dicyclohexylamine, ethanolamine, diethanolamine and triethanolamine.

The new compounds according to the invention and the physiologically acceptable salts thereof have NPY-antagonist properties and exhibit good affinities in NPY-receptor binding studies. The compounds exhibit NPY-antagonist properties both in vivo and in vitro in the pharmacological test systems described hereinafter.

In order to demonstrate the affinity of compounds according to the invention for human NPY-receptors and their antagonist properties, the following experiments were carried out:

### A. Studies of binding with SK-N-MC cells (expressing the human Y₁-receptor)

The cells are detached using a mixture of 0.02% EDTA in PBS and resuspended in 10 ml of incubation medium (MEM/25 mM Hepes + 0.5% BSA, 50 µM PMSF, 0.1% bacitracin, 3.75 mM CaCl₂) per 40 million cells approx. After 5 minutes' centrifuging (150 x g) the pellet is resuspended in the same volume and, after a further washing step in 10 ml of incubation medium, counted and diluted to 1.25 million cells/ml. Then 200 µl of a suspension of 1.25 million cells/ml is incubated for 3 hours at ambient temperature with 25 µl of a 300 pM solution of [¹²⁵I]-Bolton-Hunter-NPY and increasing concentrations (10⁻¹¹ to 10⁻⁶ M) of the test substances, whilst maintaining a total volume of 250 µl. Incubation is ended by centrifuging (10 minutes at 3000 x g and 4°C). After washing once with PBS, the radioactivity of the pellet is measured in a gamma counter. The radioactivity thus obtained represents the sum of specific and non-specific binding of [¹²⁵I]-Bolton-Hunter-NPY. The proportion of non-specific binding is defined as that radioactivity which is bound in the presence of 1 µM NPY. The IC₅₀ values of the unlabelled test substances are determined graphically. They represent the concentration of the test substance in question at which the specific binding of[¹²⁵I]-Bolton-Hunter-NPY to the NPY-Y₁ receptor is inhibited by 50%.

The following compounds of the present application have been tested according to the above described test protocol:
(A) = (R)-N-[[4-(aminocarbonylaminomethyl)phenyl]methyl]-N²-(diphenylacetyl)-argininamide,
(B) = (R,S)-N⁵-(aminoiminomethyl)-N²-(diphenylacetyl)-N-[(4-hydroxyphenyl)methyl]-N⁵-methyl-ornithinamide,
(C) = (R)-N-[[4-(aminocarbonylmethyl)phenyl]methyl]-N²-(diphenylacetyl)-argininamide,
(D) = (R)-N²-(diphenylacetyl)-N-[[4-(ethylaminocarbonylamino-methyl)phenyl]methyl]-argininamide,
(E) = (R)-N²-(diphenylacetyl)-N-[[4-(ethoxycarbonylmethylamino-carbonylaminomethyl)-phenyl)methyl]-argininamide,
(F) = (R)-N-[[4-(aminocarbonylaminomethyl)phenyl]methyl]-N²-[bis-(4-chlorophenyl)-acetyl]-argininamide,
(G) = (R)-N-[[4-(aminocarbonylaminomethyl)phenyl]methyl]-N²-[bis-(4-hydroxyphenyl)-acetyl]-argininamide,

The obtained values are shown in the following table 1:

**Table 1:**

| Compound No. | IC₅₀ [nM] |
|---|---|
| (A) | 0.37 |
| (B) | 0.62 |
| (C) | 0.63 |
| (D) | 0.75 |
| (E) | 0.4 |
| (F) | 0.35 |
| (G) | 0.17 |

### B. NPY-antagonism in vitro

Male rats (CHbb: THOM, 300 to 350 g) are given heparin (100 IU, i.v.) and the animals are then killed by a blow to the back of the neck. The abdomen is opened along the centre of the body and the left kidney is removed after the insertion of catheters in the renal artery, renal vein and ureter. The isolated kidney is immediately perfused with a modified Krebs-Ringer solution having the following composition (4 ml/minute):

| | |
|---|---|
| NaCl | 118.0 mmol/l |
| KH₂PO₄ | 1.2 mmol/l |
| KCl | 4.8 mmol/l |
| HgSO₄ | 1.2 mmol/l |
| CaCl₂ | 2.5 mmol/l |
| NaHCO₃ | 25.0 mmol/l |
| Glucose | 6.5 mmol/l |

A mixture of 95% O₂/5% CO₂ is passed through the solution which is kept at a temperature of 37°C. The perfusion pressure is measured continuously using a pressure gauge. After a 60 minute stabilisation period the perfusion rate is adjusted so as to obtain a perfusion pressure of approximately 100 mm Hg. After a further 30 minutes the experiment is started and NPY (1µM) is administered as a bolus (0.1 ml) at 15 minute intervals until the pressure increase observed reaches a constant value. The compounds to be tested are given in the form of a continuous infusion over a period of 5 minutes and then NPY is injected. After a 30 minute wash-out period the next highest concentration of test substance is investigated. 3 to 5 different concentrations of the particular compound are tested on each occasion. Concentration/activity curves can be obtained by plotting the percentage inhibition of the NPY activity against the logarithm of the concentration (mol/l) of the compound.

The obtained values are shown in the following table 2:

**Table 2**

| Compound No. | pIC₅₀ [mol/l] |
|---|---|
| (A) | 8.12 |
| (B) | 8.41 |

### C. In vivo NPY-antagonism

Male rats of normal blood pressure (Chbb:THOM, 300 to 350 g) are anaesthetised with sodium hexobarbital (150 mg/kg, i.p.). After intubation of the trachea the animals are pithed by introducing a blunt needle through the eye into the spinal bone marrow channel. The animals are ventilated with oxygen-rich ambient air using a respiratory pump (20 strokes/minute). A cannula is inserted in the left carotid artery and the arterial blood pressure is measured using a pressure gauge (Braun Melsungen Combitrans) connected to a recording device. For injection purposes a catheter is placed in the left jugular vein through which heparin is administered (200 IU/kg, i.v.). After the blood pressure has been stabilised, the animals are given 2 bolus injections of NPY (10 µg/kg, i.v.) at intervals of 15 minutes. The average increase in diastolic blood pressure is taken as the reference value (= 100%). The test substances are injected in increasing doses (4 to 6 doses) at intervals of 15 minutes. One minute after administration of the test substance, NPY is administered.

The antagonistic effect of the test substances is determined by plotting the percentage inhibition of the NPY-induced blood pressure effects against the logarithm of the concentration of active substance.

The obtained values are shown in table 3:

**Table 3:**

| Compound No. | pID₅₀ [mol/kg] |
|---|---|
| (A) | 8.11 |
| (B) | 7.87 |

### D. In vivo Studies in rats: Food intake in satiated rats

For these determinations food intake was measured in normal satiated rats after application of NPY to the paraventricular nucleus (PVN) in the presence or absence of the test compound. Male Chbb: Thom rats weighing between 300 and 350 g were used for all experiments. The rats were individually housed in stainless steel cages where water and food were available ad libitum.

Rats under Pentobarbital anaesthesia were stereotactically implanted with a stainless steel double guide cannula targeted bilaterally at the PVNs. Stereotactic coordinates were: -1.8mm caudal, 0.5mm lateral to bregma and 7.0mm below the skull surface. Injection cannulae extended the guide cannulae by 1 mm. Animals were allowed to recover from surgery for at least 10 days before being used in the experiments. Rats were handled and mock-injected during the one-week recovery period to habituate them to the injection procedure.

Animals treated with both test compounds and NPY were treated first with the test compound. Then, 15 min. after PVN infusion of the test compound or vehicle (control) 1 µg of NPY was administered by PVN infusion.

Food intake was measured by placing preweighed pellets into the cages at the time of NPY injection. Pellets were removed from the cage at 2 h post NPY injection. The food intake of animals treated with test compound was calculated as a percentage of the food intake of control animals, i.e., animals treated with vehicle.

### Food intake in food-deprived rats

Food-deprivation experiments were conducted with male Chbb:Thom rats weighing between 300 and 350 g. The animals were individually housed for the duration of the study and allowed free access to normal food together with tap water. Food was removed from the animals for 24 hours starting at 8:00 a.m. At the end of the fasting period compound or vehicle was administered to the animals by infusion into the PVN. Food was returned to the animals and their food intake monitored at various time periods during the following 24 hour period. The food intake of animals treated with test compound was calculated as a percentage of the food intake of control animals (i.e., animals treated with vehicle).

### Results:

NPY (1µg) elicited a robust feeding response in satiated rats. The injection of NPY was found to significantly induce two hour food intake, namely 3.5 g versus 0.5 g in animals injected with vehicle. The NPY induced food intake was reduced in animals treated with test compound, -e.g. with (R)-N-[[4-Aminocarbonylaminomethyl)phenyl]methyl]-N²-(diphenylacetyl)-argininamide-trifluoracetate (compound (A)).

The obtained values are shown in Table 4:

**Table 4**

| | Food intake following NPY application [g/animal] | % Inhibition of food intake |
|---|---|---|
| Vehicle | 3.53 | - |
| 15 µg of compound (A) | 2.62 | 25.9 |
| 30 µg of compound (A) | 1.67 | 52.8 |
| 60 µg of compound (A) | 1.07 | 69.8 |

It is obvious from the results of Table 4 that compound (A) produces a dose-dependent inhibition of the NPY-induced food intake. In 24 hour food deprived rats compound (A) injection (15 µg, bilaterally) resulted in a significant reduction in food intake for at least 4 h.

These experiments indicate that the compounds of the present invention inhibit food intake in rats.

In view of their pharmacological properties the compounds mentioned herein before and the physiologically acceptable salts thereof are thus suitable for the treatment of cardiovascular diseases, e.g. for treating of arterial hypertension, hypertensive crisis, stress-induced high blood pressure caused e.g. by environmental influence, physical exercise or cold stress, chronic heart insufficiency, coronary heart disease such as Angina pectoris, myocardial infarct and Syndrome X, and also for treating subarachnoidal bleeding, vascular-hypertrophic changes, e.g. restenosis after coronary angioplasty (PCTA), cerebral and coronary vasospasms, e.g. stroke, chronic kidney failure, hyperthyroidism, obesity and diabetes, epileptic diseases as well as for the diagnosis, estimation of prognosis and treatment of tumor diseases such as phaeochromocytoma, neuro(fibro)blastoma, ganglioneuroma, ganglioneuroblastoma, rhabdomyosarcoma, malignant ectomesenchymoma, anablastic astrocytoma or haemangioblastoma.

The dosage required to achieve these effects is appropriately 0.01 to 3 mg/kg of body weight, preferably 0.1 to 1 mg/kg of body weight for intravenous administration, and 0.1 to 10 mg/kg of body weight, preferably 1 to 10 mg/kg of body weight, for oral administration, in each case 1 to 3 x a day.

For this purpose, the compounds prepared according to the invention, optionally combined with other active substances such as hypotensive agents, ACE-inhibitors, diuretics and/or calcium antagonists, together with one or more inert conventional carriers and/or diluents, such as com starch, lactose, glucose, microcrystalline cellulose, magnesium stearate, polyvinylpyrrolidone, citric acid, tartaric acid, water, water/ethanol, water/glycerol, water/sorbitol, water/polyethyleneglycol, propyleneglycol, cetylstearylalcohol, carboxymethylcellulose or fatty substances such as hard fat or suitable mixtures thereof, may be made into conventional galenic preparations such as plain or coated tablets, capsules, powders, suspensions or suppositories.

For the above-mentioned combinations, the other active substances may be, for example, bendroflumethiazide, chlorothiazide, hydrochlorothiazide, spironolactone, benzothiazide, cyclothiazide, ethacrinic acid, furosemide, metoprolol, prazosine, atenolol, propranolol, (di)hydralazine-hydrochloride, diltiazem, felodipine, nicardipine, nifedipine, nisoldipine, nitrendipine, captopril, enalapril, lisinopril, cilazapril, quinapril, fosinopril and ramipril. The dosage of these active substances is preferably 1/5 of the minimum dosage normally recommended up to 1/1 of the normal recommended dosage, eg. 15 to 200 mg of hydrochlorothiazide, 125 to 2000 mg of chlorothiazide, 15 to 200 mg of ethacrinic acid, 5 to 80 mg of furosemide, 20 to 480 mg of propranolol, 5 to 60 mg of felodipine, 5 to 60 mg of nifedipine or 5 to 60 mg of nitrendipine.

The invention further relates to the use of the compounds according to the invention as valuable excipients for the production and purification (by affinity chromatography) of antibodies and, after suitable radioactive labelling, for example by direct labelling with ¹²⁵I or ¹³¹I or by tritiation of suitable precursors, for example by the replacement of halogen atoms with tritium, in RIA and ELISA assays and as a diagnostic or analytical aid in neutrotransmitter research.

The Examples which follow are intended to illustrate the invention:

### Preliminary remarks:

"Mp." denotes "melting point", "D." denotes "decomposition". Satisfactory elementary analyses, IR-, UV-, ¹H-NMR- and usually mass spectra have been obtained for all the compounds. Unless otherwise stated, R_{F} values have been determined using ready-prepared silica gel 60 F₂₅₄ TLC plates (E. Merck, Darmstadt, Item No. 5729) and an eluant consisting of n-butanol/glacial acetic acid/water = 4/1/1 (v/v/v), without chamber saturation. If there are no detailed data on the configuration, it is not specified whether it is the (R)-enantiomer or whether partial or even total racemisation has occurred.

### Example 1

### (R)-N-[[4-Aminocarbonylaminomethyl)phenyl]methyl]-N²-(diphenylacetyl)-argininamide-trifluoroacetate

### a) (R)-N⁵-[Amino(nitroimino)methyl]-N²-(diphenylacetyl)-ornithine

To a suspension of 50 g (0.228 Mol) H-D-Arg(NO₂)-OH in 400 ml of tetrahydrofuran was added a solution of 9.12 g (0.228 Mol) of sodium hydroxide in 100 ml of water. To this mixture was then added dropwise, within 30 minutes, a solution of 52.6 g (0.228 Mol) of diphenylacetylchloride in 400 ml of tetrahydrofuran and, simultaneously, a solution of 9.12 g (0.228 Mol) of sodium hydroxide in water without external cooling, the mixture was stirred for a further 12 hours at ambient temperature and then the solvents were distilled off in a water jet vacuum. The oily residue remaining was dissolved in 600 ml of water and the aqueous solution obtained was then acidified with 230 ml of IN aqueous hydrochloric acid. The precipitate formed was taken up in 500 ml of ethyl acetate, the ethyl acetate solution was then washed thoroughly with water, dried over sodium sulphate and freed from solvent in vacuo. After recrystallisation from acetone, 80.0 g (85 % of theory) of colourless crystals were obtained, Mp. 80°C.
IR (KBr): 1710, 1655 (C=O) cm⁻¹
ESI-MS: (M-H)- = 412 (calculated: 412)

### b) (R)-N-[[4-(Aminocarbonylaminomethyl)phenyl]methyl]-N⁵-[amino(nitroimino)-methyl]-N²-(diphenylacetyl)-ornithinamide

To a mixture of 5.79 g (14.0 mMol) of (R)-N⁵-[amino(nitro-imino)methyl]-N²-(diphenylacetyl)-ornithine, 2.51g (14.01 mMol) of 4-(aminocarbonylaminomethyl)-benzenemethanamine, 1.81 g (14.0 mMol) of diisopropylethylamine, 50 ml of anhydrous dimethylformamide and 25 ml of anhydrous tetrahydrofuran were added, with stirring and external cooling with ice water, 4.48 g (13.95 mMol) of TBTU. The resulting mixture was then stirred for 20 hours at ambient temperature and for 1 hour at a reaction temperature of 70°C. The solvents were distilled off in an oil pump vacuum and the residue was carefully stirred with 100 ml each of dichloromethane and water. The crystals precipitated at the interface between the two phases were suction filtered, washed with water, dichloromethane, isopropanol and diethylether and dried in vacuo. 7.72 g (96 % of theory) of a colourless crystalline substance were obtained.
IR (KBr): 1635.5 (Amide-C=O) cm⁻¹

### c) R-N-[[-4-(Aminocarbonylaminomethyl)phenyl]methyl]-N²-(diphenylacetyl)-argininamide-trifluoroacetate

A solution of 7.6 g (13.23 mMol) of (R)-N-[[4-(aminocarbonyl-aminomethyl)phenyl]-methyl]-N⁵-[amino(nitroimino)methyl]-N²-(diphenylacetyl)-ornithinamide in 200 ml of 80 percent aqueous acetic acid was hydrogenated in the presence of 4.0 g of palladium black at 40°C under 5 bar of hydrogen pressure until the uptake of hydrogen had ceased. The catalyst was filtered off, the filtrate was evaporated down in vacuo, mixed twice with 10 ml of water and once each with 10 ml of ethanol and isopropanol and evaporated down once more. The glassy residue was taken up in 200 ml of hot isopropanol, evaporated down to a volume of about 20 ml and left to stand at ambient temperature. The crystalline suspension obtained after some time was diluted with the same volume of diethylether, stirred thoroughly and suction filtered. After decoction with dichloromethane and washing with isopropanol/diethylether = 1/1 (v/v) and diethylether, 7.15 g (92 % of theory) of colourless crystals were obtained, Mp. 124-128°C, which are the acetate of the desired compound.
IR (KBr): 1649.9 (Amide-C=O) cm⁻¹
ESI-MS:(M+H)⁺ = 530
(M+Na)⁺ = 552
(M+K)⁺ = 568

If the above crystals are dissolved in 70 ml of trifluoroacetic acid and evaporated down under mild conditions in vacuo, after this process has been repeated several times, an initially oily substance is obtained which crystallises when stirred with diethylether and this is the desired trifluoroacetate.
R_{F} value: 0.56; colourless crystals, Mp. 100-105°C (D.).
IR (KBr): 1656.8 (Amide-C=O) cm⁻¹
ESI-MS: (M+H)⁺ = 530

### Example 2

### (R,S)-N⁵-(Aminoiminomethyl)-N²-(diphenylacetyl)-N-[(4-hydroxyphenyl)methyl]-N⁵-methyl]ornithinamide-hydrochloride

### a) Diethyl α-(acetylamino)-α-[3-[(phenylmethyl)methylamino]propyl]malonate

A sodium ethoxide solution freshly prepared from 5.8 g (0.252 Mol) of sodium and 250 ml of anhydrous ethanol was added dropwise at ambient temperature and within about 15 minutes to a mixture obtained from 49.4 g (0.25 Mol) of 3-chloro-N-methyl-N-(phenylmethyl)-propanamine, 60 g (0.268 Mol) of 97% diethyl acetamido-malonate, 11.3 g (0.075 Mol) of sodium iodide and 800 ml of dry dioxane. The mixture was stirred for 30 minutes at ambient temperature and then refluxed for 5 hours. It was left to stand overnight at ambient temperature, the insoluble matter was filtered off, the filtrate was freed from solvent and the residue remaining was distributed between ethyl acetate and water. The ethyl acetate phase was dried over sodium sulphate and evaporated down, the oil obtained was finally purified by column chromatography (Kieselgel MN 60, Macherey-Nagel, 70-230 mesh ASTM; mobile phase: dichloromethane/methanol/conc. aqueous ammonia = 90/10/0.25 (v/v/v)). 53 g (56 % of theory) of a colourless viscous oil were obtained.
IR (KBr): 1741.6 (Ester-C=O), 1683.8 (Amide-C=O) cm⁻¹

### b) (R,S)-N⁵-ethyl-N⁵-(phenylmethyl)-ornithine-dihydrochloride

20.4 g (0.0539 Mol) of diethylα-(acetylamino)-α-[3-[(phenylmethyl)methylamino]propyl]-malonate was dissolved in 50 ml of glacial acetic acid and after the addition of 100 ml of 3N aqueous hydrochloric acid the mixture was refluxed for 6 hours. The highly viscous, slightly yellowish mass remaining after evaporation of the solvent and obtained in quantitative yield was further reacted without any further purification.

### c) (R,S)-N²-(Diphenylacetyl)-N⁵-methyl-N⁵-(phenylmethyl)-ornithine-hydrochloride

Diphenylacetylchloride and (R,S)-N⁵-methyl-N⁵-(phenylmethyl)-ornithine-dihydrochloride were reacted analogously to Example 1a). The mixture obtained was evaporated down in a water jet vacuum until the tetrahydrofuran used as solvent had been eliminated, then acidified with 3N aqueous hydrochloric acid and extracted carefully with diethylether. The aqueous phase was then evaporated down under reduced pressure at a maximum bath temperature of +40°C. The yield of colourless crystals of Mp. 125-130°C, which were used in the next stage without purification, was 27 % of theory.
IR (KBr): 1715 (Carboxylic acid-C=O), 1664 (Amide-C=O) cm⁻¹

### d) (R,S)-N²-(Diphenylacetyl)-N-[(4-hydroxyphenyl)methyl]-N⁵-methyl-N⁵-(phenylmethyl)-ornithinamide

Prepared analogously to Example 1b) from (R,S)-N²-(diphenylacetyl)-N⁵-methyl-N⁵-(phenylmethyl)-ornithine-hydrochloride, (4-hydroxy-phenyl)methanamine and TBTU in a yield of 28% of theory.
R_{F} value: 0.75; colourless crystals, Mp. 160-162°C (ethyl acetate).
IR (KBr): 1679.9 and 1633.6 (Amide-C=O) cm⁻¹

### e) (R,S)-N²-(Diphenylacetyl)-N-[(4-hydroxyphenyl)methyl]-N⁵-methyl-ornithinamide

Prepared analogously to Example Id) from (R,S)-N²-(diphenylacetyl)-N-(4-hydroxyphenyl)-methyl]-N⁵-methyl-N⁵-(phenylmethyl)-ornithinamide by catalytic hydrogenation in the presence of palladium hydroxide/activated charcoal (Pearlman's catalyst) in a yield of 75% of theory.
R_{F} value: 0.52; colourless crystals, Mp. 118-130°C (dichloromethane).
IR (KBr): 3290 (N-H, O-H),
1635.5 (Amide-C=O) cm⁻¹
MS: M⁺ = 445

### f) (R,S)-N⁵-(Amininoiminomethyl)-N²-(diphenylacetyl)-N-[[4-hydroxyphenyl)methyl]-N⁵-methyl-ornithinamide-hydrochloride

A mixture of 0.45 g (1.01 mMol) of (R,S)-N²-(diphenylacetyl)-N-[(4-hydroxyphenyl)-methyl]-N⁵-methyl-ornithinamide, 10 ml of 1N ethanolic hydrochloric acid solution and 0.45 g (10.7 mMol) of cyanamide (97-98 percent) was refluxed for 3 days. By the addition of a little 0.1 N ethanolic hydrochloric acid solution a pH of about 5.0 was adjusted, then a further 0.15 g of cyanamide were added and the mixture was refluxed for a further 2 days. The reaction mixture was freed from solvent and the residue remaining was distributed between water and dichloromethane. The dichloromethane phase was discarded and the aqueous phase was evaporated down in vacuo. The residue was suspended in about 5 ml of water and acidified slightly with 2N aqueous hydrochloric acid to give a pH of about 3. After standing for several days, fine colourless crystals are precipitated from the aqueous solution thus obtained and these crystals are suction filtered and washed with a little water. After decoction with tetrahydrofuran and washing with acetone and diethylether, 138 mg (26% of theory) of colourless crystals were obtained, Mp. 130-135°C (D.).
R_{F} value: 0.61
IR (KBr): 1647.1 (Amide-C=O) cm⁻¹
ESI-MS:(M+H)⁺ = 488
(2M+H)⁺ = 976

### Example 3

### (R)-N-[[4-(Aminocarbonylmethyl)phenyl]methyl]-N²-(diphenyl-acetyl)-argininamide-diacetate

### a) 4-Cyanophenylacetic acid

1 g (7.03 mMol) of 4-cyanophenylacetonitrile was added to conc. hydrochloric acid preheated to 105°C and kept at this temperature for 5 minutes. It was cooled to 0°C, the precipitate formed was collected and washed thoroughly with ice water. After purification by column chromatography on silica gel (Macherey-Nagel, 35-70 mesh ASTM) using ethyl acetate/methanol/glacial acetic acid = 95/5/0.5 (v/v/v) as eluant, 0.5 g (44% of theory) of colourless crystals were obtained Mp. 152.154°C.
IR (KBr): 2229.6 (C N),
1697.2 (Carboxylic acid-C=O) cm⁻¹

### b) 4-Cyanophenylacetamide

To a solution of 1.6 g (9.93 mMol) of 4-cyanophenylacetic acid in 20 ml of anhydrous tetrahydrofuran were added, with stirring, 1.8 g (11.1 mMol) of N,N'-carbonyldiimidazole and then dry ammonia gas was introduced until a slightly alkaline reaction was obtained. The mixture was stirred for a further hour at ambient temperature, after which the solvent was distilled off in vacuo. After purification on silica gel (Baker, 0.03 to 0.06 mm) using ethyl acetate/methanol = 1/1 (v/v) as eluant and working up in the usual way, 0.7 g (44% of theory) of colourless crystals were obtained, Mp. 196-197°C (diethylether).
IR (KBr): 3447.2, 3309.0, 3203.7 (N-H),
2242.8 (C N),
1663.6 (Amide-C=O) cm⁻¹

### c) 4-(Aminocarbonylmethyl)benzenemethanamine

0.65 g (4.057 mMol) of 4-cyanophenylacetamide were dissolved in 50 ml of methanol which was saturated with ammonia at +10°C. After the addition of 0.3 g of Raney nickel the mixture was hydrogenated in an autoclave at 40°C under 5 bar of hydrogen pressure. After the uptake of hydrogen had ended the catalyst was filtered off and excess ammonia was distilled off with the solvent. The residue was acidified with 20 percent hydrochloric acid against Congo red and the non-basic impurities were removed by etherification. The ether extract was discarded, the aqueous solution was made alkaline with sodium hydroxide with external cooling and exhaustively extracted with ether. The ether solution was dried over caustic potash and freed from solvent, the residue was triturated with a few drops of ether and suction filtered. 610 mg (100% of theory) of colourless crystals were obtained, Mp. 138-140°C.
IR (KBr): 1660.6, 1637.5 (Amide-C=O) cm⁻¹

### d) (R)-N-[[4-(Aminocarbonylmethyl)phenyl)methyl]-N⁵-[amino-(nitroimino)methyl]-N²-(diphenylacetyl)-ornithinamide

Prepared analogously to Example 1b) but using dimethylformamide as solvent instead of dimethylformamide/tetrahydrofuran mixture and 4-methylmorpholine instead of diisopropylethylamine as auxiliary base, from (R)-N⁵-[amino(nitroimino)methyl]-N²-(diphenylacetyl)-ornithine, 4-(aminocarbonylmethyl)benzenemethanamine and TBTU in a yield of 73% of theory. Colourless amorphous substance.
IR (KBr): 1658.7 (Amide-CO) cm⁻¹
ESI-MS:(M+H)⁺ = 560
(M+Na)⁺ = 582

### e) (R)-N-[[4-(Aminocarbonylmethyl)phenyl]methyl]-N²-(diphenylacetyl)-argininamide-diacetate

Prepared analogously to Example 1c) from (R)-N-[[4-(aminocarbonylmethyl)phenyl]-methyl]-N⁵-[amino(nitroimino)methyl]-N²-(diphenylacetyl)-ornithine by catalytic hydrogenation in the presence of palladium black and 80 percent aqueous acetic acid in a yield of 33% of theory.
R_{F} value: 0.58; colourless crystals, Mp. 115-117°C (acetone).
IR (KBr): 1662.5 (Amide-C=O) cm⁻¹
ESI-MS: (M+H)⁺ = 515

### Example 4

### (R)-N²-(Diphenylacetyl)-N-[[4-(methylaminocarbonylaminomethyl)-phenyl]methyl]-argininamide-trifluoroacetate

### a) (R)-N²-(Diphenylacetyl)-N-[[4-(methylaminocarbonylaminomethyl)phenyl]methyl]-N^{G}-(2,2,5,7,8-pentamethylchroman-6-sulphonyl)-argininamide

To a solution of 0.75 g (0.996 mMol) of (R)-N-[[4-(aminomethyl)-phenyl]methyl]-N²-(diphenylacetyl)-N^{G}-(2,2,5,7,8-pentamethyl-chroman-6-sulphonyl)-argininamide and 0.3 ml (3,17 mMol) of triethylamine in 5 ml of anhydrous tetrahydrofuran was added a solution of 0.063 g (1.104 mMol) methylisocyanate in 1 ml of dry tetrahydrofuran. The mixture was diluted with 20 ml of diethylether, the precipitate formed was suction filtered, washed thoroughly with ether, dried, then washed with water, after which it was dried again. 0.57 g (71% of theory) of colourless crystals were obtained, Mp. 105-120°C.
IR (KBr): 1643.3 (Amide-C=O),
1298.0, 1166.9 (SO₂-N) cm⁻¹
ESI-MS:(M+H)⁺ = 810
(M+Na)⁺ = 832
(M+K)⁺ = 848

### b) (R)-N²-(Diphenylacetyl)-N-[[4-(methylaminocarbonylamino-methyl)phenyl]methyl]-argininamide-trifluoroacetate

Prepared analogously to Example If) from (R)-N²-(diphenylacetyl)-N-[[4-(methylaminocarbonylaminomethyl)phenyl]methyl]-N^{G}-(2,2,5,7,8-pentamethylchroman-6-sulphonyl)-argininamide and trifluoroacetic acid in a yield of 89% of theory. R_{F} value: 0.56; colourless crystals, Mp. 94-97°C.
IR (KBr): 1658.7 (Amide-C=O) cm⁻¹
ESI-MS:(M+H)⁺ = 544
(M+Na)⁺ = 566

### Example 5

### (R)-N²-(Diphenylacetyl)-N-[[4-(ethylaminocarbonylaminomethyl)-phenyl]methyl]-argininamide-bis-(trifluoroacetate)

### a) (R)-N²-(Diphenylacetyl)-N-[[4-(ethylaminocarbonylamino-methyl)phenyl]methyl]-N⁶-(2,2,5,7,8-pentamethylchroman-6-sulphonyl)-argininamide

Prepared analogously to Example 4a) from (R)-N-[[4-(aminomethyl)phenyl]methyl]-N²-(diphenylacetyl)-N^{G}-(2,2,5,7,8-pentamethylchroman-6-sulphonyl)-argininamide and ethylisocyanate in a yield of 93% of theory.
Colourless crystals.
IR (KBr): 1637.5 (Amide-/Urea-C=O) cm⁻¹
ESI-MS:(M+H)⁺ = 824
(M+Na)⁺ = 846
(M+K)⁺ = 862

### b) (R)-N²-(Diphenylacetyl)-N-[[4-(ethylaminocarbonylamino-methyl)phenyl]methyl]-argininamide-bis-(trifluoroacetate)

Prepared analogously to Example If) from (R)-N²-(diphenylacetyl)-N-[[4-(ethylaminocarbonylaminomethyl)phenyl]methyl]-N^{G}-(2,2,5,7,8-pentamethylchroman-6-sulphonyl)-argininamide and trifluoroacetic acid in a yield of 87% of theory.
R_{F} value: 0.68; colourless crystals.
IR (KBr): 1652.9 (Amide-/Urea-C=O) cm⁻¹
ESI-MS:(M+H)⁺ = 558
(M+Na)⁺ = 580
(M+H+Na)⁺⁺ = 290.5

### Example 6

### (R)-N-[[4-(Aminocarbonylaminomethyl)phenyl]methyl]-N²-[bis-(4-methoxyphenyl)acetyl]-argininamide-acetate

### a) (R')-N-[[4-(Aminocarbonylaminomethyl)phenyl]methyl]-N⁵-[amino(nitroimino)-methyl]-N²-[bis-(4-methoxyphenyl)acetyl]-ornithinamide

Prepared analogously to Example IVc) from bis-(4-methoxyphenyl)-acetic acid, (R)-N-[[4-(aminocarbonylaminomethyl)phenyl]methyl]-N⁵-[amino(nitroimino)methyl]-ornithinamide-hydrochloride and TBTU in a yield of 48% of theory. Colourless crystals, Mp. 149-151°C (Acetonitrile).
IR (KBr): 1635.5 (Amide-/Urea-C=O, C=N) cm⁻¹

### b) (R)-N-[[4-(Aminocarbonylaminomethyl)phenyl]methyl]-N²-[bis-(4-methoxyphenyl)-acetyl]-argininamide-acetate

Prepared analogously to Example 1c) from (R)-N-[[4-(aminocarbonylaminomethyl)phenyl]-methyl]-N⁵-[amino(nitroimino)methyl]-N²-[bis-(4-methoxyphenyl)acetyl]-ornithinamide by catalytic hydrogenation in the presence of palladium black and 80% aqueous acetic. acid in a yield of 68 % of theory.
R_{F} value: 0.58; colourless crystals.
IR (KBr): 3415.7 (N-H),
1635.5 (Amide-/Urea-C=O) cm⁻¹
ESI-MS: (M+H)⁺ = 590

### Example 7

### (R)-N²-(Diphenylacetyl)-N-[[4-(ethoxycarbonylmethylamino-carbonylaminomethyl)phenyl]-methyl]-argininamide-trifluoroacetate

### a) (R)-N²-(Diphenylacetyl)-N-[[4-(ethoxycarbonylmethylamino-carbonylaminomethyl)-phenyl]methyl]-N^{G}-(2,2,5,7,8-penta-methylchroman-6-sulphonyl)-argininamide

Prepared analogously to Example 4a) from (R)-N-[[4-(aminomethyl)phenyl]methyl]-N²-(diphenylacetyl)-N^{G}-(2,2,5,7,8-pentamethyl-chroman-6-sulphonyl)-argininamide and ethyl isocyanatoacetate in a yield of 80% of theory. Colourless, amorphous, jelly-like substance.
IR (KBr): 1739.7 (Carboxylate-C=O),
1652.9 (Amide-C=O),
1298.0, 1166.9 (SO₂-N) cm⁻¹
ESI-MS:(M+H)⁺ = 882.4
(M+Na)⁺ = 904.3
(M+2Na)⁺⁺ = 463.5

### b) (R)-N²-(Diphenylacetyl)-N-[[4-(ethoxycarbonylmethyl-aminocarbonylaminomethyl)-phenyl]methyl]-argininamide-trifluoroacetate

Prepared analogously to Example If) from (R)-N²-(diphenylacetyl)-N-[[4-(ethoxycarbonylmethylaminocarbonylaminomethyl)phenyl]methyl]-N^{G}-(2,2,5,7,8-pentamethylchroman-6-sulphonyl)-argininamide and trifluoroacetic acid in a yield of 80% of theory.
R_{F} value: 0.73; colourless crystals.
IR (KBr): 1733.9 (Carboxylate-C=O),
1654.8 (Amide-/Urea-C=O),
1203.5, 1179.1, 1134.1 (Trifluoroacetate) cm⁻¹
ESI-MS: (M+H)⁺ = 616

### Example 8

### (R)-N-[[4-(Aminocarbonylaminomethyl)phenyl]methyl]-N²-[bis-(4-fluorophenyl)acetyl]-argininamide-trifluoroacetate

### a) (R)-N-[[4-(Aminocarbonylaminomethyl)phenyl]methyl]-N²-[bis-(4-fluorophenyl)-acetyl]-N^{G}-(2,2,5,7,8-pentamethylchroman-6-sulphonyl)-argininamide

Prepared analogously to Example Xa) from bis-(4-fluorophenyl)-acetic acid, (R)-N-[[4-(aminocarbonylaminomethyl)phenyl]methyl]-N^{G}-(2,2,5,7,8-pentamethylchroman-6-sulphonyl)-argininamide and TBTU in a yield of 60% of theory.
Colourless crystals.
IR (KBr): 3435.0, 3348.2 (N-H),
1652.9 (Amide-/Urea-C=O) cm⁻¹

### b) (R)-N-[[4-(Aminocarbonylaminomethyl)phenyl]methyl]-N²-[bis-(4-fluorophenyl)-acetyl]-argininamide-trifluoroacetate

Prepared analogously to Example If) from (R)-N-[[4-(aminocarbonylaminomethyl)phenyl]-methyl]-N²-[bis-(4-fluorophenyl)acetyl]-N^{G}-(2,2,5,7,8-pentamethylchroman-6-sulphonyl)-argininamide and trifluoroacetic acid in a yield of 44% of theory.
R_{F} value: 0.60; colourless crystals, Mp. 120°C (D.).
IR (KBr): 1654.8 (Amide-/Urea-C=O) cm⁻¹
ESI-MS: (M+H)⁺ = 566

### Example 9

### (R)-N-[[4-(Aminocarbonylaminomethyl)phenyl]methyl]-N²-[bis-(4-chlorophenyl)acetyl]-argininamide-trifluoroacetate

### a) (R)-N-[[4-(Aminocarbonylaminomethyl)phenyl]methyl]-N²-[bis-(4-chlorophenyl)-acetyl]-N^{G}-(p)-argininamide

Prepared analogously to Example Xa) from bis-(4-chlorophenyl)-acetic acid, (R)-N-[[4-(aminocarbonylaminomethyl)phenyl]methyl]-N^{G}-(2,2,5,7,8-pentamethylchroman-6-sulphonyl)-argininamide and TBTU in a yield of 70% of theory. Colourless crystals, Mp. 180-183°C (Ethyl acetate).
IR (KBr): 3436.9, 3344.4 (N-H),
1629.8 (Amide-/Urea-C=O),
1299.9, 1166.9 (SO₂-N) cm⁻¹

### b) (R)-N-[[4-(Aminocarbonylaminomethyl)phenyl]methyl]-N²-[bis-(4-chlorophenyl)-acetyl]-argininamide-trifluoroacetate

Prepared analogously to Example If) from (R)-N-[[4-(aminocarbonylaminomethyl)phenyl]-methyl]-N²-[bis-(4-chlorophenyl)acetyl]-N^{G}-(2,2,5,7,8-pentamethylchroman-6-sulphonyl)-argininamide and trifluoroacetic acid in a yield of 70% of theory.
R_{F} value: 0.63; colourless crystal, Mp. 175-178°C (D.).
IR (KBr): 1652.9 (Amide-/Urea-C=O) cm⁻¹
ESI-MS: (M+H)⁺ = 598.1/600/602 (Cl₂)

### Example 10

### (R)-N-[[4-(Aminocarbonylaminomethyl)phenyl]methyl]-N²-[bis-(4-hydroxyphenyl)acetyl]-argininamide-trifluoroacetate

### a) (R)-N-[[4-(Aminocarbonylaminomethyl)phenyl]methyl]-N²-[bis-(4-hydroxyphenyl)-acetyl]-N⁷-(2,2,5,7,8-pentamethylchroman-6-sulphonyl)-argininamide

Prepared analogously to Example 1b) from bis-(4-hydroxyphenyl)-acetic acid (M. H. Hubacher, J. Org. Chem. 24, 1949 - 1951 (1959)), (R)-N-[[4-(aminocarbonylaminomethyl)-phenyl]methyl]-N⁷-(2,2,5,7,8-pentamethylchroman-6-sulphonyl)-argininamide and TBTU in a yield of 63% of theory, colourless amorphous substance.
IR (KBr): 1651.0 (Amide-, Urea-C=O),
1298.0, 1168.8 (SO₂-N) cm⁻¹

### b) (R)-N-[[4-(Aminocarbonylaminomethyl)phenyl]methyl]-N²-[bis-(4-hydroxyphenyl)-acetyl]-argininamide-trifluoroacetate

Prepared analogously to Example If) from (R)-N-[[4-(aminocarbonylaminomethyl)phenyl]-methyl]-N²-[bis-(4-hydroxyphenyl)acetyl]-N⁷-(2,2,5,7,8-pentamethylchroman-6-sulphonyl)-argininamide and trifluoroacetic acid in a yield of 76 % of theory.
R_{F} value: 0.43; colourless, amorphous substance.
IR (KBr): 1658.7 (Amide-, Urea-C=O),
1201.6, 1182.8, 1137.9 (Trifluoroacetate) cm⁻¹
ESI-MS:(M+H)⁺ = 562
(M+Na)⁺ = 584

### Example 11

### (R)-N-[[4-(Aminocarbonylaminomethyl)phenyl]methyl]-N²-[bis-[4-(methoxycarbonylmethoxy)phenyl]acetyl]-argininamide-acetate

### a) (R)-N-[[4-(Aminocarbonylaminomethyl)phenyl]methyl]-N²-[(1,1-dimethylethoxy)-carbonyl]-N⁷-nitroargininamide

Prepared analogously to Example VIe) from (R)-N²-[(1,1-dimethylethoxy)carbonyl]-N⁷-nitroarginine and 4-(aminocarbonylaminomethyl)benzenemethanamine in the presence of TBTU in a yield of 54% of theory.
Colourless crystals, Mp. 173-175°C (D.).
IR (KBr): 1681.8 (Carbamate-C=O),
1660.6 (Amide-/Urea-C=O),
1525.6, 1315.4 (NNO₂) cm⁻¹

### b) (R)-N-[[4-(Aminocarbonylaminomethyl)phenyl]methyl]-N⁷-nitroargininamide

Prepared analogously to Example IXi) from (R)-N-[[4-(aminocarbonylaminomethyl)phenyl]-methyl]-N²-[(1,1-dimethylethoxy)carbonyl]-N⁷-nitroargininamide by treating with methanolic hydrogen chloride solution and subsequently converting into the base in a quantitative yield.
Colourless crystals (Isopropanol).
IR (KBr): 1652.9 (Amide-/Urea-C=O) cm⁻¹
ESI-MS:(M+H)⁺ = 381
(M+Na)⁺ = 403
(M+K)⁺ = 419

### c) (R)-N-[[4-(Aminocarbonylaminomethyl)phenyl]methyl]-N²-[bis-(4-hydroxyphenyl)-acetyl]-N⁷-nitroargininamide

Prepared analogously to Example IVc) from bis(4-hydroxyphenyl)acetic acid and (R)-N-[[4-(aminocarbonylaminomethyl)phenyl)methyl)-N⁷-nitroargininamide in the presence of TBTU in a yield of 29% of theory.
Colourless, amorphous substance.

### d) (R)-N-[[4-(Aminocarbonylaminomethyl)phenyl]methyl]-N²-[bis-[4-(methoxycarbonylmethoxy)phenyl]acetyl]-N⁷-nitroargininamide

To a solution of 91 mg (3.958 mMol) of sodium in 30 ml of anhydrous methanol were added, successively, 1.2 g (1.978 mMol) of (R)-N-[[4-(aminocarbonylaminomethyl]phenyl]methyl]-N²-[bis-(4-hydroxyphenyl)acetyl]-N⁷-nitroargininamide and 0.392 ml (0.633 g; 4.14 mMol) of methyl bromoacetate and the mixture was kept for 24 hours at a reaction temperature of 45°C. After working up the usual way and purifying by column chromatography (silica gel MN 60, Macherey-Nagel, 70 - 230 mesh ASTM; mobile phase: dichloromethane/methanol/cyclohexane/conc. aqueous ammonia = 68/15/15/2 (v/v/v/v)) 0.11 g (7.4% of theory) of a colourless amorphous substance were obtained.
R_{F} value: 0.43(Polygram® SIL G/UV₂₅₄ ready-made sheets for TLC made by Macherey-Nagel, Art.-No. 805021; eluant: dichloromethane/methanol/cyclohexane/conc. aqueous ammonia = 68/15/15/2 (v/v/v/v)).
MS: M⁺ = 750

### e) (R)-N-[[4-(Aminocarbonylaminomethyl)phenyl]methyl]-N²-[bis-[4-(methoxycarbonylmethoxy)phenyl]acetyl]-argininamide-acetate

Prepared analogously to Example 1c) from (R)-N-[[4-(aminocarbonylaminomethyl)phenyl]-methyl]-N²-[bis-[4-(methoxycarbonylmethoxy)phenyl]acetyl]-N⁷-nitroargininamide by catalytic hydrogenation in the presence of palladium black and 80% aqueous acetic acid in a yield of 90% of theory.
R_{F} value: 0.37; colourless, amorphous substance.
IR (KBr): 1751.3 (Carboxylate-C=O),
1658.7 (Amide-/Urea-C=O) cm⁻¹
ESI-MS: (M+H)⁺ = 706.3

### Example 12

### (R)-N-[[4-(Aminocarbonylaminomethyl)phenyl]methyl]-N²-[bis-[4-(hydroxycarbonylmethoxy)phenyl]acetyl]-argininamide-acetate

### a) (R)-N-[[4-(Aminocarbonylaminomethyl)phenyl]methyl]-N²-[bis-[4-(hydroxycarbonylmethoxy)phenyl]acetyl]-N⁷-nitroargininamide

Prepared analogously to Example VIIa) from (R)-N-[[4-(aminocarbonylaminomethyl)-phenyl]methyl]-N²-[bis-[4-(methoxycarbonylmethoxy)phenyl]acetyl]-N⁷-nitroargininamide by saponification with 1N aqueous sodium hydroxide solution in a yield of 98% of theory.
Colourless, amorphous substance.
ESI-MS:(M-H)⁻ =721.1
(M-2H)²⁻ = 360.2
(M-2H+Na)⁻ =743.1

### b) (R)-N-[[4-(Aminocarbonylaminomethyl)phenyl]methyl]-N²-[bis-[4-(hydroxycarbonylmethoxy)phenyl]acetyl]-argininamide-acetate

Prepared analogously to Example 1c) from (R)-N-[[4-(aminocarbonylaminomethyl)phenyl]-methyl]-N²-[bis-[4-(hydroxycarbonylmethoxy)phenyl]acetyl]-N⁷-nitroargininamide by catalytic hydrogenation in the presence of palladium black and 80% aqueous acetic acid in a yield of 79% of theory.
R_{F} value: 0.18; colourless, amorphous substance.
IR (KBr): 1718.5 (Carboxylic acid-C=O),
1654.8 (Amide-/Urea-C=O) cm⁻¹
ESI-MS:(M+H)⁺ = 678
(M-H)⁻ = 676
(M-2H)²⁻ = 337.7

### Example I

### (R)-N-[[4-Acetylaminomethyl)phenyl]methyl]-N²-(diphenylacetyl)argininamide-trifluoroacetate

### a) (R)-N²-(9-Fluorenylmethoxycarbonyl)-N^{G}-(2,2,5,7,8-penta-methylchroman-6-sulphonyl)-N-[[4-[[(phenylmethoxycarbonyl)-amino]methyl]phenyl]methyl]-argininamide

To a solution of 15.0 g (20.14 mMol) Fmoc-D-Arg(Pmc)-OH (89 percent) in 250 ml of anhydrous tetrahydrofuran were added, under the protection of inert gas, at ambient temperature and with stirring, one after the other, 4.33 g (20.99 mMol) of dicyclohexylcarbodiimide, 2.83 g (20.94 mMol) of HOBT and 5.68 g (21.01 mMol) of 4-[[(phenylmethoxycarbonyl)amino]methyl]benzenemethanamide (R. Epton et al., Polymer 21: 481-482 (1980)), the mixture was stirred for a further hour at ambient temperature then for 1 hour at 60°C and finally overnight at ambient temperature. The insoluble matter was filtered off, the solvent was eliminated from the filtrate, the initially amorphous residue was intensively stirred with 200 ml of dichloromethane and after the addition of 600 ml of water to the resulting solution it was shaken for 30 minutes in a shaking machine. The resulting precipitate was suction filtered and purified by decocting with 300 ml of diethylether and intensively washing several times with dichloromethane and ether. After drying in vacuo, 14.50 g (79 % of theory) of colourless crystals were obtained, Mp. 132-136°C (D.).
IR (KBr): 1693.3 (Urethane-CO),
1652.9, 1625.9 (Amide-CO, C=N) cm⁻¹
ESI-MS: (M+H)⁺ = 915
(M+Na)⁺ = 937

### b) (R)-N^{G}-(2,2,5,7,8-Pentamethylchroman-6-sulphonyl)-N-[[4-[[(phenylmethoxycarbonyl)amino]methyl]phenyl]methyl)-argininamide

A solution of 13.5 g (14.75 mMol) of (R)-N²-(9-fluorenylmethoxycarbonyl)-N^{G}-(2,2,5,7,8-pentamethylchroman-6-sulphonyl)-N-[[4-[[(phenylmethoxycarbonyl)amino]methyl]phenyl]-methyl]-argininamide in 80 ml of dimethylformamide was mixed with 18 ml (175 mMol) of diethylamine, shaken thoroughly and left to stand for 4 hours at ambient temperature. The solvent was eliminated in an oil pump vacuum at slightly elevated temperature, the residue was distributed between ethyl acetate and water, the combined ethyl acetate phases were dried over sodium sulphate and evaporated down in vacuo. The amorphous, glassy residue remaining was purified by column chromatography on silica gel (Macherey-Nagel, 35-70 mesh ASTM) using initially dichloromethane/methanol/conc. aqueous ammonia = 90/10/0.25 and subsequently dichloromethane/methanol/conc. aqueous ammonia = 80/20/0.5 (v/v/v) as eluant. By evaporating the appropriate fractions down, 9.8 g (96% of theory) of the desired compound were obtained as a colourless, amorphous/glassy substance.
IR (KBr): 1714.6 (Urethane-CO),
1620.1 cm⁻¹ (C=N)

### c) (R)-N²-(Diphenylacetyl)-N^{G}-(2,2,5,7,8-pentamethylchroman-6-sulphonyl)-N-[[4-[[(phenylmethoxycarbonyl)amino]methyl]-phenyl]methyl]-argininamide

Prepared analogously to Example Ia) from diphenylacetic acid and (R)-N^{G}-(2,2,5,7,8-pentamethylchroman-6-sulphonyl)-N-[[4-[[(phenylmethoxycarbonyl)amino]methyl]phenyl]-methyl]-argininamide in the presence of dicyclohexylcarbodiimide and hydroxybenzotriazole in a yield of 96% of theory. Colourless crystals, Mp. 118-121°C (D.).
IR (KBr): 3442.7, 3307.7 (NH),
1693.4 (Urethane-CO),
1643.3 (Amide-CO) cm⁻¹

### d) (R)-N-[[4-(Aminomethyl)phenyl]methyl]-N²-(diphenylacetyl)-N^{G}-(2,2,5,7,8-pentamethylchroman-6-sulphonyl)-argininamide

A mixture of 11.75 g (13.25 mMol) of (R)-N²-(diphenylacetyl)-N^{G}-(2,2,5,7,8-pentamethylchroman-6-sulphonyl)-N-[[4-[[(phenyloxycarbonyl)amino]methyl]phenyl]methyl]arginin-amide, 300 ml methanol and 2.0 g of 10 percent palladium on animal charcoal was hydrogenated at ambient temperature under 5 bars of hydrogen pressure until the uptake of hydrogen had ceased. The catalyst was filtered off, the filtrate was evaporated down in vacuo, the amorphous/glassy residue remaining was purified on silica gel (Macherey-Nagel, 0.063 to 0.2 mm) by column chromatography, using dichloromethane/methanol/conc. aqueous ammonia = 80/20/0.25 (v/v/v) as eluant. By evaporating the appropriate fractions down, 7.88 g (79% of theory) of the desired compound were obtained as a colourless,
glassy/amorphous substance.
IR(KBr): 1652.9 (Amide-CO) cm⁻¹

### e) (R)-N-[[4-(Acetylaminomethyl)phenyl]methyl]-N²-(diphenyl-acetyl)-N^{G}-(2,2,5,7,8-pentamethylchroman-6-sulphonyl)-argininamide

To a solution of 1.0 g (1.328 mMol) of (R)-N-[[4-(aminomethyl)phenyl]methyl]-N²-(diphenylacetyl)-N^{G}-(2,2,5,7,8-pentamethylchroman-6-sulphonyl)-argininamide in 10 ml of ethanol was added dropwise within 10 minutes a solution of 0.15 g (1.469 mMol) of acetic anhydride in 3 ml of diethylether. The mixture was evaporated down in vacuo, the residue was distributed between dichloromethane and saturated aqueous potassium carbonate solution. The dichloromethane phase was dried over sodium sulphate and freed from solvent, the residue was digested again with a little dichloromethane and suction filtered after crystallising out. After drying in vacuo, 0.91 g (86% of theory) of colourless crystals were obtained, Mp. 128-132°C.
IR(KBr): 1643.3 (Amide-CO) cm⁻¹

### f) (R)-N-[[4-(Acetylaminomethyl)phenyl]methyl]-N²-(diphenyl-acetyl)-argininamide-trifluoroacetate

Into a mixture of 18.6 ml of trifluoroacetic acid, 0.6 ml of anisole, 0.4 ml of 1,2-ethanedithiol and 0.4 ml of water which is externally cooled with ice/methanol, were added within 20 minutes and with stirring 0.85 g (1.069 mMol) of (R)-N-[[4-(acetylaminomethyl)phenyl]-methyl]-N²-(diphenylacetyl)-N^{G}-(2,2,5,7,8-pentamethylchroman-6-sulphonyl)-argininamide. After the cooling was removed the mixture was stirred for a further 14 hours at ambient temperature. It was filtered, the filtrate was diluted with the same volume of diethylether and filtered again. The filtrate thus obtained was evaporated down in vacuo and the residue was digested intensively several times with diethylether. The crystals obtained were finally suction filtered, washed with diethylether and dried over phosphorus pentoxide in vacuo. 0.67 g (98% of theory) of colourless crystals were obtained.
R_{f} value: 0.57
IR (KBr): 1652.9 (Amide-CO),
1203.5, 1185.8, 1136.0 (Trifluoroacetate) cm⁻¹
ESI-MS:(M+H)⁺ = 529
(2M+H)⁺ = 1057

### Example II

### (R)-N²-(Diphenylacetyl)-N-[[4-(ethoxycarbonylaminomethyl)-phenyl]methyl]-argininamide-trifluoroacetate

### a) (R)-N²-(Diphenylacetyl)-N-[[4-(ethoxycarbonylaminomethyl)-phenyl)methyl]-N^{G}-(2,2,5,7,8-pentamethylchroman-6-sulphonyl)argininamide

To a solution of 1.0 g (1.328 mMol) of (R)-N-[[4-(aminomethyl)phenyl]methyl]-N²-(diphenylacetyl)-N^{G}-(2,2,5,7,8-pentamethylchroman-6-sulphonyl)-argininamide in 10 ml of anhydrous tetrahydrofuran were added dropwise first 0.2 g (1.976 mMol) of triethylamine, then a solution of 0.16g (1.474 mMol) of ethylchlorocarbonate in 2 ml of anhydrous tetrahydrofuran. After 10 minutes' stirring at ambient temperature the mixture was evaporated down in vacuo, the residue was distributed between water and dichloromethane, the dichloromethane phase was dried over sodium sulphate and freed from solvent in vacuo. The residue remaining was intensively triturated with ether and suction filtered. 1.0 g (91% of theory) of a colourless product were obtained, Mp. 117-120°C.
IR (KBr): 1689.5 (Carbonate-C=O),
1643.3 (Amide-C=O) cm⁻¹

### b) (R)-N²-(Diphenylacetyl-N-[[4-(ethoxycarbonylaminomethyl)phenyl]methyl]-argininamide-trifluoroacetate

Prepared analogously to Example If) from (R)-N²-(diphenylacetyl-N-[[4-(ethoxycarbonyl-aminomethyl)phenyl]methyl]-N⁶-(2,2,5,7,8-pentamethylchroman-6-sulphonyl)-argininamide and trifluoroacetic acid in a yield of 86% of theory.
R_{f} value: 0.72; colourless substance, Mp. 76-80°C (D.).
IR (KBr): 1668.3 (Amide-C=O) cm⁻¹
ESI-MS:(M+H)⁺ = 559
(2M+H)⁺ =1117

### Example III

### (R)-N-[[3-(Aminocarbonylaminomethyl)phenyl]methyl]-N²-(diphenylacetyl)argininamide-acetate

### a) 3-(Aminocarbonylaminomethyl)benzenemethanamine

A mixture of 4.08 g (29.96 mMol) of 3-(aminomethyl)benzene-methanamine, 30 ml tetrahydrofuran and 30 ml 1N aqueous hydrochloric acid were mixed with 1.95 g (29.58 mMol) of sodium cyanate added in batches. After stirring for 6 hours at ambient temperature the solvents were largely distilled off in vacuo, the residue was distributed between water and dichloromethane, the dichloromethane phase was dried over sodium sulphate and evaporated down. The residue was purified by column chromatography on silica gel (Macherey-Nagel, 35-70 mesh ASTM) using dichloromethane/methanol/cyclohexane/conc. aqueous ammonia = 68/15/15/2 (v/v/v/v) as eluant. By working up the corresponding fractions, 0.9 g (17% of theory) of colourless crystals were obtained, Mp. 127-129°C (diethylether).
IR (KBr): 3379.3, 3311.6 (N-H),
1658.7 (Urea-C=O) cm⁻¹

### b) (R)-N-[[3-(Aminocarbonylaminomethyl)phenyl]methyl]-N⁵-[amino(nitroimino]-methyl]-N²-(diphenylacetyl)-ornithinamide

Prepared analogously to Example 1b) from (R)-N⁵-[amino(nitroimino]methyl]-N²-(diphenyl-acetyl)-ornithine, 3-(aminocarbonylaminomethyl)benzenemethanamine and TBTU in a yield of 50% of theory. Colourless amorphous substance.
IR (KBr): 1651.0 (Amide-/Urea-C=O) cm⁻¹
ESI-MS: (M+H)⁺ = 575
(M+Na)⁺ = 597
(M+K)⁺ = 613

### c) (R)-N-[[3-(Aminocarbonylaminomethyl)phenyl]methyl]-N²-(diphenylacetyl)-argininamide-acetate

Prepared analogously to Example 1c) from (R)N-[[3-(aminocarbonylaminomethyl)phenyl]-methyl]-N⁵-[amino(nitroimino]methyl]-N²-(diphenylacetyl)-ornithinamide by catalytic hydrogenation in the presence of palladium black and 80% acetic acid in a yield of 42% of theory.
R_{f} value: 0.61; colourless crystals, Mp. 98-103°C.
IR (KBr): 1641.3 (Amide-/Urea-C=O) cm⁻¹
ESI-MS:(M+H)⁺ = 530
(M+Na)⁺ = 5552.

### Example IV

### (R,S)-N-[[[4-(Aminocarbonylaminomethyl)phenyl]methyl]-3-[3-(aminoiminomethyl)-phenyl]-N²-(diphenylacetyl)-alaninamide acetate

### a) (R,S)-3-(3-Cyanophenyl)alanine-hydrochloride

Prepared analogously to Example 2b) from diethyl α-(acetamido)-α-[(3-cyanophenyl)-methyl]malonate (Mp. 139-141°C; prepared analogously to Example 2a) from diethyl α-acetamido-malonate and 3-(bromomethyl)benzonitrile in the presence of sodium methoxide), hydrochloric acid and glacial acetic acid in a yield of 69% of theory.
Colourless crystals, Mp. 206°C (D.).

### b) (R,S)-3-(3-Cyanophenyl)-N²-(diphenylacetyl)-alanine

Prepared analogously to Example 1a) from (R,S)-3-(3-cyanophenyl)-alanine-hydrochloride and diphenylacetylchloride in the presence of sodium hydroxide solution in a yield of 58% of theory.
Colourless crystals, Mp. 145-147°C (ethyl acetate).
IR(KBr): 3380 (N-H),
2230 (C N),
1725 (Carboxylic acid-C=O),
1665 (Amide-C=O),
1515 (Amide-II) cm⁻¹

### c) (R,S)-N-[[4-(Aminocarbonylaminomethyl)phenyl]methyl]-3-(3-cyanophenyl)-N²-(diphenylacetyl)-alaninamide

Prepared analogously to Example 1b) but using dimethylformamide instead of dimethylformamide/tetrahydrofuran mixture, from (R,S)-3-(3-cyanophenyl)-N²-(diphenylacetyl)-alanine, 4-(aminocarbonylaminomethyl)benzenemethanamine and TBTU in a yield of 59% of theory. Colourless crystals, Mp. 210-212°C (ethanol).
IR (KBr): 2229.6 (C N),
1641.3 (Amide/Urea-C=O) cm⁻¹

### d) (R,S)-N-[[4-(Aminocarbonylaminomethyl)phenyl]methyl]-3-[3-[amino(hydroxyimino)-methyl]phenyl]-N²-(diphenylacetyl)-alaninamide

A mixture of 273 mg (0.5 mMol) of (R,S)-N-[[4-(aminocarbonylaminomethyl)phenyl]-methyl]-3-(3-cyanophenyl)-N²-(diphenylacetyl)-alaninamide, 69.5 mg (1.0 mMol) of hydroxylaminohydrochloride, 30 ml of methanol and 0.17 ml (0.126 g; 1 mMol) of diisopropylethylamine were refluxed for 24 hours. The same amounts of hydroxylaminehydrochloride and diisopropylethylamine were added again and the mixture was refluxed for a further 24 hours. It was filtered hot and the solvent was eliminated from the filtrate. The residue was stirred with water and suction filtered. The colourless crystals obtained were decocted with acetone, washed with ether and dried.
Yield: 190 mg (66% of theory).
IR (KBr): 1641.3 (Amide/Urea-C=O)
ESI-MS:(M+H)⁺ = 579
(M+Na)⁺ = 601

### e) (R,S)-N-[[4-(Aminocarbonylaminomethyl)phenyl]methyl]-3-[3-aminoiminomethyl)-phenyl]-N²-(diphenylacetyl)-alanine-amide-acetate

Prepared analogously to Example 1c), but using palladium on animal charcoal (10%) as catalyst and using glacial acetic acid as solvent, from (R,S)-N-[[4-(aminocarbonylaminomethyl)phenyl]methyl]-3-[3-[amino(hydroxyimino)methyl]phenyl]-N²-(diphenylacetyl)-alaninamide in a yield of 38% of theory.
R_{f} value: 0.66; colourless crystals, Mp. 188-190°C.
IR (KBr): 1643.3 (Amide-/Urea-C=O) cm⁻¹,
Bands of salt
ESI-MS: (M+H)⁺ = 563

### Example V

### (R)-N²-(Diphenylacetyl)-N-[[4-(methoxycarbonylmethyl)phenyl]-methyl]-argininamide-diacetate

### a) Methyl 4-cyanobenzeneacetate

20.1 g (0.125 Mol) of 4-cyanobenzeneacetate were dissolved in 400 ml of dichloromethane and, after the addition of 59.5 g (0.5 Mol) of thionylchloride, refluxed for 4 hours. The excess thionylchloride was distilled off together with the solvent, finally in vacuo, the residue was taken up in 400 ml of dry methanol and refluxed for 1 hour. The crude product remaining after the excess methanol had been distilled off was purified by column chromatography on silica gel (Macherey-Nagel, 0.2-0.5 mm) using petroleum ether/ethyl acetate = 8/2 (v/v) as eluant and, after the usual working up of the suitable fractions, yielded 9.5 g (43% of theory) of colourless needles, Mp. 40-41°C (petroleum ether/diisopropylether 1/1, (v/v)).
IR(KBr): 2227.7(CN),
1735.8 (Carboxylate-C=O) cm⁻¹

### b) 4-(Methoxycarbonylmethyl)benzenemethanamine-hydrochloride

A solution of 8.8 g (0.05 Mol) of methyl 4-cyanobenzene acetate in 200 ml of methanol was hydrogenated, after the addition of 50 ml of 1 N aqueous hydrochloric acid and. 3 g of palladium on activated charcoal (10%), at ambient temperature under a hydrogen pressure of 3 bar until the uptake of hydrogen had ended. The solution freed from catalyst was evaporated down, the residue was combined with two batches of 50 ml of toluene and evaporated down again. 10.7 g (99% of theory) of a crude crystalline material was obtained which was used without further purification in the next step.

### c) (R)-N⁵-[Amino(nitroimino)methyl]-N²-(diphenylacetyl)-N-[[4-(methoxycarbonylmethyl)phenyl]methyl]-ornithinamide

Prepared analogously to Example 3d) from (R)-N⁵-[amino(nitroimino)-methyl]-N²-(diphenylacetyl)-ornithinamide and 4-(methoxycarbonylmethyl)benzenemethanamine-hydrochloride in a yield of 21% of theory. Colourless crystals, Mp. 158-160°C.
IR (KBr): 1739.7 (Carboxylate-C=O),
1641.3 (Amide-C=O) cm⁻¹

### d) (R)-N²-(Diphenylacetyl)-N-[[4-(methoxycarbonylmethyl)-phenyl]methyl]-argininamide-diacetate

Prepared analogously to Example 1c) from (R)-N⁵-[amino(nitroimino)methyl]-N²-(diphenyl-acetyl)-N-[[4-(methoxycarbonylmethyl)phenyl]methyl]-ornithinamide by catalytic hydrogenation in the presence of palladium black and 80% aqueous acetic acid in a yield of 44% of theory.
R_{f} value: 0.67; colourless, amorphous substance.
IR (KBr): 1737.8 (Carboxylate-C=O),
1652.9 (Amide-C=O) cm⁻¹:
ESI-MS:(M+H)⁺ = 530
(M+Na)⁺ = 552

### Example VI

### (R)-[[4-[[[(Dimethylamino)carbonyl]methylamino]methyl]phenyl]-methyl]-N²-(diphenylacetyl)-argininamide-trifluoroacetate

### a) 4-Cyano-N-methyl-N-(phenylmethyl)benzenemethanamine

To a solution of 12.9 ml (0.1 Mol) of benzylmethylamine in 50 ml of tetrahydrofuran were added, in batches 9.8 g (0.05 Mol) of 4-(bromomethyl)benzonitrile and the mixture was then stirred for 6 hours at ambient temperature and 3 hours at a reaction temperature of 30°C. The mixture was filtered, the filtrate was evaporated down in vacuo, the residue was taken up in 50 ml of diethylether, filtered again and the filtrate was evaporated down once more. A quantitative yield of an oil was obtained which was used in the next step without further purification.
IR (KBr): 2227.7 (C N) cm⁻¹

### b) 4-[[(Phenylmethyl)methylamino]methyl]benzenemethanamine

To a suspension of 1.9 g (0.05 Mol) of lithium aluminium hydride in 70 ml of anhydrous tetrahydrofuran was added dropwise, at ambient temperature, a solution of 12.1 g (0.051 Mol) of 4-cyano-N-methyl-N-(phenylmethyl)benzenemethanamine in 30 ml of dry tetrahydrofuran and the mixture was then heated to 60°C for 3 hours and refluxed for 2 hours. A further 0.5 g of lithium aluminium hydride were added and the mixture was refluxed for another 3 hours. After working up in the usual way and purification by column chromatography (Baker; 0.03-0.06 mm; dichloromethane/methanol/cyclohexane/conc. aqueous ammonia = 68/15/15/2 (v/v/v/v)) 10.2 g (83% of theory) of a colourless oil were obtained.

### c) (R)-N²-(Fmoc)-N^{G}-(2,2,5,7,8-pentamethylchroman-6-sulphonyl)-N-[[4-[[(phenylmethyl)methylamino]methyl]phenyl]methyl]-argininamide

Prepared analogously to Example Ia) from (R)-N²-(Fmoc)-N^{G}-(2,2,5,7,8-pentamethylchroman-6-sulphonyl)-arginine,4-[[(phenylmethyl)methylamino]methyl]benzenemethanamine and dicyclohexylcarbodiimide in a quantitative yield.
Colourless, amorphous substance.
IR (KBr): 1724.3 (Carbamate-C=O),
1662.5, 1618.2(Amide-C=O, C=N),
1369.4, 1107.1 (SO₂-N) cm⁻¹
ESI-MS:(M+H)⁺ = 885
(M+Na)⁺ = 907

### d) (R)-N^{G}-(2,2,5,7,8-pentamethylchroman-6-sulphonyl)-N-[[4-[[(phenylmethyl)methylamino]methyl]phenyl]methyl]-argininamide

Prepared analogously to Example Ib), but using tetrahydrofuran as solvent instead of dimethylformamide, from (R)-N²-(Fmoc)-N^{G}-(2,2,5,7,8-pentamethylchroman-6-sulphonyl)-N-[[4-[[(phenylmethyl)memylamino]methyl]phenyl]methyl]-argininamide and diethylamine in a yield of 88% of theory.
Colourless, amorphous substance.
IR (KBr): 3435.0, 3336.7 (N-H),
1618.2 (Amide-C=O, C=N),
1298.0, 1166.9 (SO₂-N) cm⁻¹

### e) (R)-N²-(Diphenylacetyl)-N^{G}-(2,2,5,7,8-pentamethylchroman-6-sulphonyl)-N-[[4-[[(phenylmethyl)methylamino]methyl]phenyl]methyl]-argininamide

Prepared analogously to Example Ib), but using tetrahydrofuran as solvent instead of dimethylformamide/tetrahydrofuran mixture, from diphenylacetic acid, (R)-N^{G}-(2,2,5,7,8-pentamethylchroman-6-sulphonyl)-N-[[4-[[(phenylmethyl)methylamino]methyl]phenyl]-methyl]-argininamide and TBTU in a yield of 99% of theory.
Colourless, amorphous substance.
IR (KBr): 3433.1, 3323.2 (N-H),
1620.1, 1651.0 (Amide-C=O, C=N),
1382.9, 1166.9 (SO₂-N) cm⁻¹

### f) (R)-N²-(Diphenylacetyl)-N-[[4-[(methylamino)methyl]phenyl]-methyl]-N^{G}-(2,2,5,7,8-pentamethylchroman-6-sulphonyl)-argininamide

Prepared analogously to Example Id), but using ethanol as solvent instead of methanol, from (R)-N²-(diphenylacetyl)-N^{G}-(2,2,5,7,8-pentamethylchroman-6-sulphonyl)-N-[[4-[[(phenylmethyl)methylamino]methyl]phenyl]methyl]-argininamide by catalytic hydrogenation in the presence of palladium/activated charcoal in a yield of 34% of theory.
Colourless, amorphous substance.
IR (KBr): 3431.2, 3321.2 (N-H),
1651.0 (Amide-C=O, C=N),
1298.0, 1166.9 (SO₂-N) cm⁻¹

### g) (R)-N-[[4-[[[(Dimethylamino)carbonyl]methylamino]methyl]-phenyl]methyl]-N²-(diphenylacetyl)-N^{G}-(2,2,5,7,8-penta-methylchroman-6-sulphonyl)-argininamide

Prepared analogously to Example IIa) from (R)-N²-(diphenylacetyl)-N-[[4-[(methylamino)-methyl]phenyl]methyl]-N^{G}-(2,2,5,7,8-pentamethylchroman-6-sulphonyl)-argininamide and dimethylcarbamoylchloride in a yield of 84% of theory.
Colourless, crystalline substance.
IR (KBr): 1622.0 (Amide-C=O, C=N) cm⁻¹

### h) (R)-N-[[4-[[[(Dimethylamino)carbonyl]methylamino]methyl]-phenyl]methyl]-N²-(diphenylacetyl)-argininamide-trifluoroacetate

Prepared analogously to Example If) from (R)-N-[[4-[[[(dimethylamino)carbonyl]methylamino]methyl]phenyl]methyl]-N²-(diphenylacetyl)-N^{G}-(2,2,5,7,8-pentamethylchroman-6-sulphonyl)-argininamide and trifluoroacetic acid in a yield of 89% of theory.
R_{f} value: 0.64; colourless crystals.
IR (KBr): 1668.3 (Amide-C=O),
1203.5, 1176.5, 1130.2 (Trifluoroacetate) cm⁻¹
ESI-MS:(M+H)⁺ = 572
(M+Na)⁺ = 594

### Example VII

### (R)-N-[[4-[[[(Carboxymethyl)amino]carbonyl]methyl]phenyl]-methyl]-N²-(diphenylacetyl)-argininamide-diacetate

### a) (R)-N⁵-[Amino(nitroimino)methyl]-N-[[4-(carboxymethyl)-phenyl]methyl]-N²-(diphenylacetyl)-ornithinamide

440 mg (0.766 mMol) of (R)-N⁵-[Amino(nitroimino)methyl]-N²-(diphenylacetyl)-N-[[4-(methoxycarbonylmethyl)phenyl]methyl]-omithinamide were dissolved in 50 ml of methanol and after the addition of 2.4 ml (2.4 mMol) of 1N sodium hydroxide solution the mixture was refluxed for 3 hours. The methanol was distilled off under reduced pressure, the residue was diluted with 5 ml of water and carefully acidified with 1N hydrochloric acid. It was exhaustively extracted with ethyl acetate, the combined ethyl acetate extracts were dried over sodium sulphate and evaporated down. The residue was stirred several times with a little diisopropylether/diethylether and after drying yielded 400 mg (93% of theory) of a colourless, amorphous substance.
IR (KBr): 1710.8 (Carboxylic acid-C=O),
1641.3 (Amide-C=O) cm⁻¹

### b) (R)-N⁵-[Amino(nitroimino)methyl]-N²-(diphenylacetyl)-N-[[4-[[(methoxycarbonylmethyl)amino]carbonyl]methyl]phenyl]-methyl]-ornithinamide

Prepared analogously to Example 3d) from (R)-N⁵-[amino(nitroimino)-methyl]-N-[[4-(carboxymethyl)phenyl]methyl]-N²-(diphenylacetyl)-ornithinamide, glycinemethylester hydrochloride and TBTU in a yield of 52% of theory.
Colourless crystals, Mp. 168-170°C.
IR (KBr): 1757.0 (Carboxylate-C=O),
1645.2 (Amide-C=O) cm⁻¹

### c) (R)-N⁵-[Amino(nitroimino)methyl]-N-[[4-[[[(carboxymethyl)-amino]carbonyl]methyl]-phenyl]methyl]-N²-(diphenylacetyl)-ornithinamide

Prepared analogously to Example VIIa) by saponification of (R)-N⁵-[amino(nitroimino)-methyl]-N²-(diphenylacetyl)-N-[[4-[[[(methoxycarbonylmethyl)amino]carbonyl]methyl]-phenyl]methy]-ornithinamide in a yield of 80% of theory. Colourless crystals, Mp. 160-162°C.
IR (KBr): 3377.2, 3311.6, 3274.9 (N-H, O-H),
1637.5 (Amide-C=O) cm⁻¹

### d) (R)-N-[[4-[[[(Carboxymethyl)amino]carbonyl]methyl]phenyl]-methyl]-N²-(diphenylacetyl)-argininamide-diacetate

Prepared analogously to Example 1c) from N⁵-[amino(nitroimino)methyl]-N-[[4-[[[(carboxymethyl)amino]carbonyl]methyl]phenyl]methyl]-N²-(diphenylacetyl)ornithinamide by catalytic hydrogenation in the presence of palladium black and 80% aqueous acetic acid in a yield of 65% N² of theory.
R_{f} value: 0.49; amorphous-glassy substance.
IR (KBr): 1652.9 (Amide-C=O) cm⁻¹
ESI-MS:(M+H)⁺ =573
(M+Na)⁺ = 595

### Example VIII

### (R)-N-[[4-(Aminocarbonylaminomethyl)phenyl]methyl]-N²-(diphenylacetyl)-N-(ethoxycarbonylmethyl)-argininamide-diacetate

### a) Ethyl [[(4-cyanophenyl)methyl]amino]acetate

A mixture of 27.9 g (0.2 Mol) of glycine ethylester-hydrochloride, 350 ml of methanol, 3.9 g (0.062 Mol) of sodium cyanoborohydride and 13.1 g (0.1 Mol) of 4-cyanobenzaldehyde was stirred for 26 hours at ambient temperature, then evaporated to dryness in vacuo. The residue was distributed between ethyl acetate and saturated potash solution, the organic phase was dried over sodium sulphate and evaporated down in vacuo. The residue was purified on silica gel (Macherey-Nagel, 35-70 mesh ASTM) using petroleum ether/ethyl acetate = 1/1 (v/v) as eluant and after the appropriate fractions have been worked up 11.7 g (54% of theory) of a colourless oil were obtained.
IR(KBr): 3340 (N-H),
2230 (C N),
1735 (Carboxylate-C=O) cm⁻¹

### b) N²-[(4-Cyanophenyl)methyl]-N²-[(1,1-dimethylethoxy)carbonyl]-glycineethylester

A solution of 11.7 g (0.054 Mol) of ethyl [[(4-cyanophenyl)methyl]amino]acetate in 200 ml of anhydrous tetrahydrofuran was mixed with 13.1 g (0.06 Mol) di-tert.butyl-pyrocarbonate. The mixture was stirred for 3 hours at ambient temperature, then evaporated down in vacuo and 16.8 g (98% of theory) of a slightly yellowish oil were obtained which were used in the following step without any further purification.
IR (KBr): 2229.6 (C N),
1749.3 (Carboxylate-C=O),
1703.0 (Urethane-C=O) cm⁻¹

### c) N²-[[4-(Aminomethyl)phenyl]methyl]-N²-[(1,1-dimethylethoxy)carbonyl]-glycine-ethylester-hydrochloride

Prepared analogously to Example Vb) from N²-[(4-cyanophenyl)methyl]-N²-[(1,1-dimethylethoxy)carbonyl]-glycineethylester by catalytic hydrogenation in the presence of palladium/activated charcoal and 1 equivalent of 1N hydrochloric acid in a yield of 88% of theory.
Colourless, amorphous substance which was used in the next step without any further purification.
IR (KBr): 1749.3 (Carboxylate-C=O),
1701.1 (Urethane-C=O) cm⁻¹

### d) N²-[[4-(Aminocarbonylaminomethyl)phenyl]methyl]-N²-[(1,1-dimethylethoxy)-carbonyl]-glycineethylester

Prepared analogously to Example IIIa) from N²-[[4-(aminomethyl)-phenyl]methyl]-N²-[(1,1-dimethylethoxy)carbonyl]-glycineethyl-ester-hydrochloride and sodium cyanate in a yield of 93% of theory. Colourless, amorphous substance which was used in the next step without any further purification.
IR (KBr): 1749.3 (Carboxylate-C=O),
1697.3 (Urethane-C=O),
1664.5 (Urea-C=O) cm⁻¹

### e) N²-[[4-(Aminocarbonylaminomethyl)phenyl]methyl]-glycine-ethylester-trifluoracetate

A solution of 1.8 g (4.93 mMol) of N²-[[4-(aminocarbonylaminomethyl)phenyl]methyl]-N²-[(1,l-dimethylethoxy)carbonyl]-glycineethylester in 56 ml of dichloromethane was mixed with a total of 3.4 ml of trifluoroacetic acid, which was added dropwise thereto whilst externally cooling with ice, and the resulting mixture was then stirred overnight at room temperature. The excess trifluoroacetic acid was removed together with the solvent by vacuum distillation, the residue was taken up several times in a little dichloromethane and evaporated down again and finally 0.7 g (37% of theory) of colourless crystals were obtained, Mp. 90-91°C.
IR (KBr): 3440.8, 3336.7 (N-H),
1739.7 (Carboxylate-C=O),
1652.9 (Urea-C=O) cm⁻¹,
Trifluoroacetate bands

### f) (R)-N-[[4-(Aminocarbonylaminomethyl)phenyl]methyl]-N⁵-[amino(nitroimino)-methyl]-N²-(diphenylacetyl)-N-(ethoxycarbonylmethyl)-ornithinamide

Prepared analogously to Example IVc) from (R)-N⁵-[amino[nitroimino)methyl]-N²-(diphenylacetyl)-ornithine, N²-[[4-(aminocarbonylaminomethyl)phenyl]methyl]-glycineethylester-trifluoroacetate and TBTU in a yield of 42% of theory.
Colourless, amorphous substance.
IR (KBr): 1741.6 (Carboxylate-C=O),
1645.2 (Amide-/Urea-C=O) cm⁻¹
ESI-MS:(M+H)⁺ =661
(M+Na)⁺ =683
(M+K)⁺ = 699

### g) (R)-N-[[4-(Aminocarbonylaminomethyl)phenyl]methyl]-N²-(diphenylacetyl)-N-(ethoxycarbonylmethyl)-argininamide-diacetate

Prepared analogously to Example 1c) from (R)-N-[[4-(aminocarbonylaminomethyl)phenyl]-methyl]-N⁵-[amino(nitroimino)methyl]-N²-(diphenylacetyl)-N-(ethoxycarbonylmethyl)-ornithinamide by catalytic hydrogenation in the presence of palladium black and 80% aqueous acetic acid in a yield of 90% of theory.
R_{f} value: 0.64; glassy-amorphous substance.
IR (KBr): 1745.5 (Carboxylate-C=O),
1656.8 (Amide-/Urea-C=O) cm⁻¹
ESI-MS: (M+H)⁺ = 616

### Example IX

### (R)-N-[[2-(Aminocarbonyl)-2,3-dihydro-1H-isoindol-5-yl]methyl]-N²-(diphenylacetyl)-argininamide

### a) Methyl 2,3-dihydro-2-(phenylmethyl)-1H-isoindole-5-carboxylate

A mixture of 77.0 g (0.469 Mol) of methyl 3,4-dimethylbenzoate, 178.0 g (1.0 Mol) of N-bromosuccinimide, 0.5 g of azoisobutyronitrile and 800 ml of tetrachloromethane was refluxed for 1 hour whilst simultaneously being subjected to intensive illumination with a 1000-Watt daylight bulb. The mixture was allowed to cool to about 40°C, then filtered and the filter residue was washed thoroughly with 200 ml of tetrachloromethane. Within about 30 minutes, at a reaction temperature of +30°C, a mixture of 53.6 g ( 0.575 Mol) of benzenemethanamine, 101.2 g (1.0 Mol) of triethylamine and 150 ml of toluene was added to the combined filtrates. The resulting mixture was refluxed for 3 hours, then left overnight at ambient temperature and filtered to remove the precipitate formed. The filtrate was freed from the solvent in vacuo, the residue remaining was distributed between tert.butyl-methylether and 20% aqueous citric acid, then the aqueous phase was extracted thoroughly with tert.butyl-methylether and ethyl acetate. Batches of sodium hydrogen carbonate were added to the aqueous phase until the development of carbon dioxide ceased, then the mixture was exhaustively extracted with a tert.butyl-methylether/ethyl acetate mixture (1:1 v/v). The combined extracts were worked up in the usual way and yielded 48.1 g (38% of theory) of colourless crystals, Mp. 72°C.
IR (CH₂Cl₂): 1735 (Carboxylate-C=O) cm⁻¹

### b) 2,3-Dihydro-2-(phenylmethyl)-1H-isoindole-5-carboxylic acid-hydrochloride

Prepared analogously to Example VIIa) by saponification of methyl 2,3-dihydro-2-(phenylmethyl)-lH-isoindole-5-carboxylate in a quantitative yield. By treating with conc. Hydrochloric acid the compound was converted into its hydrochloride, which was used in the following step without any further purification.
IR (KBr): 1710, 1695 (Carboxylic acid-C=O) cm⁻¹

### c) 2,3-Dihydro-2-(phenylmethyl)-1H-isoindole-5-carboxylic acid chloride-hydrochloride

40.0 g (0.138 Mol) of 2,3-Dihydro-2-(phenylmethyl)-1H-isoindole-5-carboxylic acid-hydrochloride were suspended in 400 ml of anhydrous tetrahydrofuran and at a reaction temperature of 50 to 55°C 100 g (0.84 Mol) of thionylchloride were added dropwise. The temperature was maintained at 50 to 60°C until the development of gas had ceased completely and then the cooled solution was filtered to remove the insoluble matter. The filtrate was evaporated down in vacuo, the residue was triturated with 5 ml of dry tetrahydrofuran, suction filtered and dried over diphosphorus pentoxide in a desiccator. 34.8 g (82% of theory) of colourless crystals were obtained, Mp. 226-228°C (D.).
IR (KBr): 1793, 1755 (Carboxylic acid chloride-C=O) cm⁻¹

### d) 2,3-Dihydro-2-(phenylmethyl)-1H-isoindole-5-carboxamide

To a mixture of 200 ml of conc. aqueous ammonia and 50 ml of tetrahydrofuran were added, in batches, 20.0 g (64.9 mMol) of 2,3-dihydro-2-(phenylmethyl)-1H-isoindole-5-carboxylic acid chloride-hydrochloride and the mixture was stirred overnight at ambient temperature. The precipitate formed was suction filtered, washed thoroughly with water and dried at 50°C in a circulating air dryer. The desired compound was obtained in a quantitative yield in the form of colourless crystals.
IR (KBr): 3370, 3170 (N-H),
1650 (Amide-C=O) cm⁻¹

### e) 2,3-Dihydro-2-(phenylmethyl)-1H-isoindole-5-ylmethanamine

Prepared analogously to Example VIb) from 2,3-dihydro-2-(phenylmethyl)-1H-isoindole-5-carboxamide by reduction with lithium aluminium hydride. The desired compound was obtained as a colourless oil in a yield of 74% of theory.

### f) 2,3-Dihydro-5-[[[(1,1-dimethylethoxy)carbonyl]amino]methyl]-2-(phenylmethyl)-1H-isoindole

Prepared analogously to Example VIIIb) from 2-(phenylmethyl)-2,3-dihydro-1H-isoindol-5-ylmethanamine and di-tert.butylpyrocarbonate in a yield of 34% of theory. The product was used in the following step without further purification.

### g) 2.3-Dihydro-5-[[[(1,1-dimethylethoxy)carbonyl]amino]methyl]-1H-isoindole

Prepared analogously to Example Id), but using tetrahydrofuran as solvent instead of methanol and with the addition of 1 equivalent of 1N aqueous hydrochloric acid, from 2,3-dihydro-5-[[[(1,1-dimethylethoxy)carbonyl]amino]methyl]-2-(phenylmethyl)-1H-isoindole by catalytic hydrogenation in the presence of palladium/activated charcoal in a yield of 97% of theory. Colourless crystals, Mp. 114 - 115°C.
IR (KBr): 3450 (N-H),
1710 (Carbamate-C=O) cm⁻¹

### h) 2-(Aminocarbonyl)-2,3-dihydro-5-[[[(1,1-dimethylethoxy)-carbonyl]amino]methyl]-1H-isoindole

Prepared analogously to Example IIIa) from 2,3-dihydro-5-[[[(1,1-dimethylethoxy)carbonyl]-amino]methyl]-1H-isoindole, 1 equivalent of 1N hydrochloric acid and sodium cyanate in a quantitative yield.
Colourless crystals.
IR (KBr): 3392.6 (N-H),
1647.1, 1606.6 (C=O) cm⁻¹
MS: M⁺=291

### i) 2-(Aminocarbonyl)-2,3-dihydro-1H-isoindol-5-yl-methanamine hydrochloride

A solution of 2.2 g (7.55 mMol) of 2-(aminocarbonyl)-2,3-dihydro-5-[[[(1,1-dimethylethoxy)carbonyl]amino]methyl]-1H-isoindole in 20 ml of saturated methanolic hydrogen chloride solution was stirred for 1 hour at ambient temperature, then freed in vacuo from excess hydrogen chloride and the solvent. The residue was triturated with tetrahydrofuran and suction filtered and finally dried overnight in the desiccator.
Yield: 94% of theory; colourless crystals, Mp. 222-223°C.
IR (KBr): 1662.5 (Amide-C=O) cm⁻¹

### j) (R)-N-[[2-(Aminocarbonyl)-2,3-dihydro-1H-isoindol-5-yl]-methyl]-N⁵-[amino(nitroimino)methyl]-N²-(diphenylacetyl)-ornithinamide

Prepared analogously to Example 1b), but using dimethylsulphoxide as solvent instead of dimethylformamide/tetrahydrofuran mixture and with the addition of HOBt, from (R)-N⁵-[amino[nitroimino)methyl]-N²-(diphenylacetyl)-ornithinine and 2-(aminocarbonyl)-2,3-dihydro-1H-isoindol-5-yl-methanamine-hydrochloride in a yield of 21% of theory.
Colourless crystals.
IR (KBr): 3288.4 (N-H),
1641.3 (Amide-C=O) cm⁻¹

### k) (R)-N-[[2-(Aminocarbonyl)-2,3-dihydro-1H-isoindol-5-yl]-methyl]-N²-(diphenylacetyl)-argininamide

Prepared analogously to Example 1c) from (R)-N-[[2-(aminocarbonyl)-2,3-dihydro-1H-isoindol-5-yl]methyl]-N⁵-[amino(nitroimino)methyl]-N²-(diphenylacetyl)-ornithinamide by catalytic hydrogenation in the presence of palladium black and 80% aqueous acetic acid in a yield of 65% of theory.
R_{f} value: 0.30; amorphous-foamy substance.
IR (KBr): 1641.3 (Amide-C=O) cm⁻¹
ESI-MS:(M+H)⁺ = 542
(M+Na)⁺ = 564

### Example X

### (R)-N-[[4-(Aminocarbonylaminomethyl)phenyl]methyl]-N²-[[(1-naphthyl)]amino]carbonyl]-argininamide-acetate

### a) (R)-N-[[4-(Aminocarbonylaminomethyl)phenyl]methyl]-N⁵-[amino(nitroimino)-methyl]-N²-[(1,1-dimethylethoxy)-carbonyl]-ornithinamide

Prepared analogously to Example IVc), but with the addition of HOBt, from (R)-N⁵-[amino-(nitroimino)methyl]-N²-[(1,1-dimethylethoxy)-carbonyl]-ornithine, 4-(aminocarbonylaminomethyl)-benzenemethanamine and TBTU in a yield of 80% of theory.
Colourless crystals, Mp. 172°C.
IR (KBr): 3454.3, 3419.6, 3332.8 (N-H),
1681.8 (Carbamate-C=O),
1660.6 (Amide-/Urea-C=O) cm⁻¹

### b) (R)-N-[[4-(Aminocarbonylaminomethyl)phenyl]methyl]-N⁵-[amino(nitroimino)-methyl]-ornithinamide-hydrochloride

Prepared analogously to Example IXi) from (R)-N-[[4-(aminocarbonylaminomethyl)phenyl]-methyl]-N⁵-[amino(nitroimino)methyl]-N²-[(1,1-dimethylethoxy)carbonyl]-ornithinamide and methanolic hydrogen chloride solution in a quantitative yield.
Colourless crystals.
IR (KBr): 1676.0, 1635.5 (Amide-/Urea-C=O) cm⁻¹
ESI-MS:(M+H)⁺ = 381
(M+Na)⁺ = 403

### c) (R)-N-[[4-(Aminocarbonylaminomethyl)phenyl]methyl]-N⁵-[amino(nitroimino)-methyl]-N²-[[(1-naphthyl)amino]carbonyl]-ornithinamide

Prepared analogously to Example 4a), but using dimethylformamide as solvent instead of tetrahydrofuran and diisopropylethylamine as auxiliary base instead of triethylamine, from 1-naphthylisocyanate and (R)-N-[[4(aminocarbonylaminomethyl)phenyl]methyl]-N⁵-[amino-(nitroimino)methyl]-omithinamide-hydrochloride in a yield of 71% of theory.
Colourless crystals, Mp. 210°C.
IR (KBr): 1625.9 (Amide-/Urea-C=O) cm⁻¹
ESI-MS:(M+H)⁺ = 550
(M+Na)⁺ = 572
(M+K)⁺ = 588

### d) (R)-N-[[4-(Aminocarbonylaminomethyl)phenyl]methyl]-N²-[[(1-naphthyl)amino]-carbonyl]-argininamide-acetate

Prepared analogously to Example 1c) from (R)-N-[[4-(aminocarbonylaminomethyl)phenyl]-methyl]-N⁵-[amino(nitroimino)methyl]-N²-[[(1-naphthyl)amino]carbonyl]-ornithinamide by catalytic hydrogenation in the presence of palladium black and 80% aqueous acetic acid in a yield of 58% of theory.
R_{f}value: 0.31; colourless crystals.
IR (KBr): 3333.2 (N-H),
1648.1, 1628.9 (Amide-/Urea-C=O),
1546.4 (Amide-II) cm⁻¹
ESI-MS:(M+H)⁺ = 505
(M+Na)⁺ = 527

## Claims

1. A compound selected from the group consisting of:
(1) (R)-N-[[4-(aminocarbonylaminomethyl)phenyl]methyl]-N²-(diphenylacetyl)-argininamide,
(2) (R,S)-N⁵-(aminoiminomethyl)-N²-(diphenylacetyl)-N-[(4-hydroxyphenyl)methyl]-N⁵-methyl-ornithinamide,
(3) (R) -N- [[4-(aminocarbonylmethyl)phenyl] methyl]-N²-(diphenylacetyl)-argininamide,
(4) (R)-N²-(diphenylacetyl)-N-[[4-(methylaminocarbonyl-aminomethyl)phenyl]methyl] -argininamide,
(5) (R)-N²-(diphenylacetyl)-N-[[4-(ethylaminocarbonylaminomethyl)phenyl]methyl]-argininamide,
(6) (R)-N-[[4-(aminocarbonylaminomethyl)phenyl]methyl]-N²-[bis-(4-methoxyphenyl)acetyl]-argininamide,
(7) (R)-N²-(diphenylacetyl)-N-[[4-(ethoxycarbonylmethylaminocarbonylaminomethyl)phenyl]methyl]-argininamide,
(8) (R)-N-[[4-(aminocarbonylaminomethyl)phenyl]methyl]-N²-[bis-(4-fluorophenyl)acetyl]-argininamide,
(9) (R)-N-[[4-(aminocarbonylaminomethyl)phenyl]methyl]-N²-[bis-(4-chlorophenyl)acetyl]-argininamide,
(10) (R)-N-[[4-(aminocarbonylaminomethyl)phenyl]methyl]-N²-[bis-(4-hydroxyphenyl)acetyl]-argininamide,
(11) (R)-N-[[4-(aminocarbonylaminomethyl)phenyl]methyl]-N²-[bis-[4-(methoxycarbonylmethoxy)phenyl]acetyl]-argininamide and
(12) (R)-N-[[4-(aminocarbonylaminomethyl)phenyl]methyl]-N²-[bis-[4-(hydroxycarbonylmethoxy)phenyl]acetyl]-argininamide
or a salt thereof.

2. Physiologically acceptable salts of the compounds of claim 1 with inorganic or organic acids or bases.

3. A pharmaceutical composition comprising a compound of claim 1 or a physiologically acceptable salt thereof according to claim 2.

4. Use of a compound of claim 1 or a physiologically acceptable salt thereof according to claim 2 for preparing a pharmaceutical composition according to claim 3 which is suitable for the treatment of cardiovascular diseases, coronary heart diseases, subarachnoidal bleeding, vascular-hypertrophic changes, cerebral or coronary vasospasm, chronic kidney failure, hyperthyroidism, obesity, diabetes and epileptic diseases and for the diagnosis, estimation of prognosis and treatment of tumour diseases.

5. Process for preparing a pharmaceutical composition according to claim 3, **characterised in that** a compound according to claim 1 or 2 is incorporated in one or more inert carriers and/or diluents by a non-chemical method.

6. Use of a compound of claim 1 or 2 in vitro as an adjuvant for the production and purification of antibodies.

7. Use of a compound of claim 1 or 2 for radioactive labelling for use in RIA or ELISA assays.

## Patentansprüche

1. Eine Verbindung, ausgewählt aus der Gruppe bestehend aus:
(1) (R)-N-[[4-(Aminocarbonylaminomethyl)phenyl]methyl]-N²-(diphenylacetyl)-argininamid,
(2) (R,S)-N⁵-(Aminoiminomethyl)-N²-(diphenylacetyl)-N-[(4-hydroxyphenyl)methyl]-N⁵-methyl-ornithinamid,
(3) (R)-N-[[4-(Aminocarbonylmethyl)phenyl)methyl]-N²-(diphenylacetyl)-argininamid,
(4) (R)-N²-(Diphenylacetyl)-N-[[4-(methylaminocarbonyl-aminomethyl)phenyl]methyl]-argininamid,
(5) (R)-N²-(diphenylacetyl)-N-[[4-(ethylaminocarbonylaminomethyl)phenyl]methyl]-argininamid,
(6) (R)-N-[[4-(Aminocarbonylaminomethyl)phenyl]methyl]-N²-[bis-(4-methoxyphenyl)acetyl]-argininamid,
(7) (R)-N²-(Diphenylacetyl)-N-([4-(ethoxycarbonylmethylaminocarbonylaminomethyl)phenyl]methyl]-argininamid,
(8) (R)-N-[[4-(Aminocarbonylaminomethyl)phenyl]methyl]-N²-[bis-(4-fluorophenyl)acetyl]-argininamid,
(9) (R)-N-[[4-(Aminocarbonylaminomethyl)phenyl]methyl]-N²-[bis-(4-chlorophenyl)acetyl]-argininamid,
(10) (R)-N-[[4-(Aminocarbonylaminomethyl)phenyl]methyl]-N²-[bis-(4-hydroxyphenyl)acetyl]-argininamid,
(11) (R)-N-[[4-(Aminocarbonylaminomethyl)phenyl]methyl]-N²-[bis-[4(methoxycarbonylmethoxy)phenyl]acetyl]-argininamid und
(12) (R)-N-[[4-(Aminocarbonylaminomethyl)phenyl]methyl]-N²-[bis[4(hydroxycarbonylmethoxy)phenyl]acetyl]-argininamid
oder ein Salz davon.

2. Physiologisch annehmbare Salze der Verbindungen nach Anspruch 1 mit anorganischen oder organischen Säuren oder Basen.

3. Eine pharmazeutische Zusammensetzung, umfassend eine Verbindung nach Anspruch 1 oder ein physiologisch annehmbares Salz davon nach Anspruch 2.

4. Verwendung einer Verbindung nach Anspruch 1 oder eines physiologisch annehmbaren Salzes davon nach Anspruch 2 zur Herstellung einer pharmazeutischen Zusammensetzung nach Anspruch 3, welche zur Behandlung von kardiovaskulären Erkrankungen, koronaren Herzkrankheiten, subarachnoidalen Blutungen, vaskulär-hypertrophischen Veränderungen, zerebralen oder koronaren Vasospasmen, chronischer Nierenisuffizienz, Hyperthyreose, Adipositas, Diabetes und epileptischen Erkrankungen sowie zur Diagnose, Abschätzung der Prognose und Behandlung von Tumorerkrankungen geeignet ist.

5. Verfahren zur Herstellung einer pharmazeutischen Zusammensetzung nach Anspruch 3, **dadurch gekennzeichnet, dass** eine Verbindung nach Anspruch 1 oder 2 mittels einer nicht-chemischen Methode in einen oder mehrere inerte Träger und/oder Verdünnungsmittel eingebaut wird.

6. Verwendung einer Verbindung nach Anspruch 1 oder 2 in vitro als Adjuvans zur Herstellung und Reinigung von Antikörpern.

7. Verwendung einer Verbindung nach Anspruch 1 oder 2 zur radioaktiven Markierung zur Verwendung in RIA oder ELISA Assays.

## Revendications

1. Composé sélectionné dans le groupe consistant en :
(1) (R)-N-[[4-(aminocarbonylaminométhyl)phényl]méthyl]-N²-(diphénylacétyl)-argininamide,
(2) (R,S)-N⁵-(aminoiminométhyl)-N²-(diphénylacétyl)-N-[(4-hydroxyphényl)méthyl]-N⁵-méthyl-ornithinamide,
(3) (R)-N-[[4-(aminocarbonylméthyl)phényl]méthyl]-N²-(diphénylacétyl)-argininamide,
(4) (R)-N²-(diphénylacétyl)-N-[[4-(méthylaminocarbonyl-aminométhyl)phényl]méthyl]-argininamide,
(5) (R)-N²-(diphénylacétyl)-N-[[4-(éthylaminocarbonylaminométhyl)phényl]méthyl]-argininamide,
(6) (R)-N-[[4-(aminocarbonylaminométhyl)phényl]méthyl]-N²-[bis-(4-méthoxyphényl)acétyl]-argininamide,
(7) (R)-N²-(diphénylacétyl)-N-[[4-(éthoxycarbonylméthylamiocarbonylaminométhyl)phényl]méthyl]-argininamide,
(8) (R)-N-[[4-(aminocarbonylaminométhyl)phényl]méthyl]-N²-[bis-(4-fluorophényl)acétyl]-argininamide,
(9) (R)-N-[[4-(aminocarbonylaminométhyl)phényl]méthyl]-N²-[bis-(4-chlorophényl)acétyl]-argininamide,
(10) (R)-N-[[4-(aminocarbonylaminométhyl)phényl]méthyl]-N²-[bis-(4-hydroxyphényl)acétyl]-argininamide,
(11) (R)-N-[[4-(aminocarbonylaminométhyl)phényl]méthyl]-N²-[bis-[4-(méthoxycarbonylméthoxy)phényl]acétyl]-argininamide et
(12) (R)-N-[[4-(aminocarbonylaminométhyl)phényl]méthyl]-N²-[bis-[4-(hydroxycarbonylméthoxy)phényl]acétyl]-argininamide
ou l'un de leurs sels.

2. Sels physiologiquement acceptables des composés de la revendication 1 avec des acides ou des bases inorganiques ou organiques.

3. Composition pharmaceutique comprenant un composé de la revendication 1 ou un sel physiologiquement acceptable de celui-ci selon la revendication 2.

4. Utilisation d'un composé de la revendication 1 ou d'un sel physiologiquement acceptable de celui-ci selon la revendication 2 pour la préparation d'une composition pharmaceutique selon la revendication 3 qui est adaptée au traitement des maladies cardiovasculaires, des maladies coronariennes, des saignements sous-arachnoïdiens, des changements vasculo-hypertrophiques, de l'angiospasme cérébral ou coronarien, de la déficience rénale chronique, de l'hyperthyroïdie, de l'obésité, des diabètes et des maladies épileptiques et au diagnostic, à l'estimation du pronostic et au traitement des maladies tumorales.

5. Procédé de préparation d'une composition pharmaceutique selon la revendication 3, **caractérisé en ce qu'**un composé selon la revendication 1 ou 2 est incorporé à un ou plusieurs véhicules et/ou diluants inertes selon un procédé non chimique.

6. Utilisation d'un composé de la revendication 1 ou 2 in vitro comme adjuvant pour la production et la purification des anticorps.

7. Utilisation d'un composé de la revendication 1 ou 2 pour le marquage radioactif pour les dosages RIA et ELISA.
